(19) Europäisches Patentamt
European Patent Office
Office européen des brevets

(11) EP 2 556 062 B1

(12) FASCICULE DE BREVET EUROPEEN

(45) Date de publication et mention de la délivrance du brevet:
**04.01.2017 Bulletin 2017/01**

(21) Numéro de dépôt: **11717310.4**

(22) Date de dépôt: **06.04.2011**

(51) Int Cl.:
***A61Q 17/04*** *(2006.01)* ***A61K 8/49*** *(2006.01)*
***C07D 333/38*** *(2006.01)* ***A61K 31/381*** *(2006.01)*
***A61P 17/00*** *(2006.01)*

(86) Numéro de dépôt international:
**PCT/FR2011/050772**

(87) Numéro de publication internationale:
**WO 2011/124844 (13.10.2011 Gazette 2011/41)**

# (54) DÉRIVÉS AMIDES DE COMPOSÉS D'ACIDE AVÉNALUMIQUE, LEUR PROCÉDÉ DE PRÉPARATION ET COMPOSITIONS COSMÉTIQUES LES CONTENANT

AMIDDERIVATE VON AVENALUMINSÄURE, VERFAHREN ZU DEREN HERSTELLUNG UND KOSMETIKZUSAMMENSETZUNG DAMIT

AMIDE DERIVATIVES OF AVENALUMIC ACID, PROCESS FOR PREPARING SAME AND COSMETIC COMPOSITIONS CONTAINING SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.04.2010 FR 1052650**

(43) Date de publication de la demande:
**13.02.2013 Bulletin 2013/07**

(73) Titulaire: **Produits Chimiques Auxiliaires et de Synthese**
**91160 Longjumeau (FR)**

(72) Inventeurs:
- **RAULT, Sylvain**
  **F-14370 Moult (FR)**
- **LANCELOT, Jean Charles**
  **F-14400 Tour en Bessin (FR)**
- **EL KIHEL, Laïla**
  **F-14530 Luc sur Mer (FR)**
- **BAZIN, Marc-Antoine**
  **F-44110 Saint Aubin des Châteaux (FR)**
- **PECQUET, Régis**
  **F-91540 Mennecy (FR)**
- **BROIN, Etienne**
  **F-92340 Bourg la Reine (FR)**

(74) Mandataire: **Zimmermann, Alain**
**Cabinet Malemont**
**91 avenue Kléber**
**75116 Paris (FR)**

(56) Documents cités:
**EP-A1- 0 227 431 JP-A- 9 059 139**

- **M.-A. BAZIN, L.EL KIHEL, M. JOUANNE, J.-C. LANCELOT, S. RAULT: "synthesis of new avelumic carboxamide derivatives in the ferulic series", SYNTHETIC COMMUNICATIONS, vol. 38, no. 22, 2008, pages 3947-3959, XP009135860,**
- **HITOSHI OINUMA ET AL: "SYNTHESIS AND BIOLOGICAL EVALUATION OF SUBSTITUTED BENZENESULFONAMIDES AS NOVEL POTENT MEMBRANE-BOUND PHOSPHOLIPASE A2 INHIBITORS", JOURNAL OF MEDICINAL CHEMISTRY, AMERICAN CHEMICAL SOCIETY, WASHINGTON, US LNKD-DOI:10.1021/JM00111A048, vol. 34, no. 7, 1 juillet 1991 (1991-07-01), pages 2260-2267, XP00602078, ISSN: 0022-2623**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 556 062 B1

## Description

La présente invention a pour objet de nouveaux dérivés des acides cinnamique, férulique et avénalumique, leur procédé de préparation et leur utilisation en cosmétologie pour la préparation de compositions cosmétiques.

On connait déjà des composés de ce type présentant des propriétés antioxydantes, de piégeage de radicaux libres, photoprotectrices et/ou anti-inflammatoires, pouvant trouver des applications en cosmétologie.

En particulier, dans la demande de brevet européen EP 0 227 431, on a déjà proposé des composés dérivés de l'acide cinnamique et de l'acide férulique possédant des propriétés anti-inflammatoires dues à l'inhibition de la phospholipase A2 et de la 5-lipoxygénase. Lesdits composés sont donc proposés pour le traitement de maladies en vue d'éviter la formation de médiateurs inflammatoires (voir P. 3, 2$^{e}$ alinéa).

La demanderesse a maintenant conçu de nouveau dérivés amides d'acide avénalumique, de structure originale leur conférant des propriétés intéressantes, à savoir des propriétés apaisantes associées à des propriétés antiradicalaires, antioxydantes et protectrices contre les UV, de telles propriétés en faisant des principes actifs de choix dans le domaine cosmétologique.

Les nouveaux dérivés selon l'invention répondent plus précisément à la formule générale :

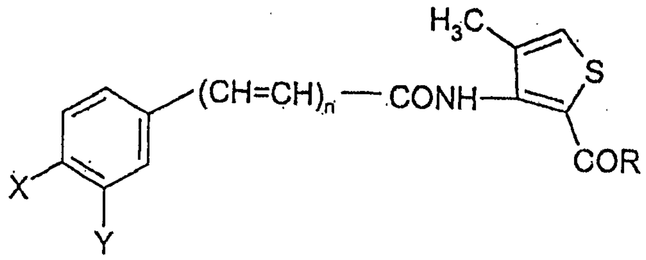

(I)

dans laquelle :

- n = 2,
- le couple (X,Y) est le couple ($OH,OCH_3$),
- R représente un groupe OH, un groupe OR' où R' est un groupe alkyle linéaire ou ramifié, en $C_1$-$C_{12}$, ou un groupe $NR_1R_2$ où $R_1$ et $R_2$ sont chacun indépendamment un atome d'hydrogène, un groupe alkyle linéaire ou ramifié, en $C_1$-$C_6$, ou un groupe cycloalkyle en $C_3$-$C_8$ ramifié ou non.

Selon un mode de réalisation, le paramètre R de la formule (I) a, de préférence, les significations suivantes :

(a) - R=OH, $OCH_3$, $OC_2H_5$, O-n$C_3H_7$, O-iso$C_3H_7$,O-n$C_4H_9$, O-iso$C_4H_9$, O-tert$C_4H_9$ ou $O(CH_2)_{11}$-$CH_3$ ; ou
(b) - R=$NR_1R_2$ où le couple (R1,R2) = (H,H), ($H,CH_3$), (H,n$C_3H_7$), (H,iso$C_3H_7$), (H,n$C_9H_9$), (H,iso$C_4H_9$) ou (H,tert$C_4H_9$).

La présente invention s'étend à tous les isomères et sels ayant la même formule structurelle que celle définie par la formule (I) ci-dessus, lesdits sels étant obtenus des dérivés de formule (I) avec une base minérale (par exemple NaOH) ou organique appropriée.

Les nouveaux dérivés, isomères et sels selon l'invention peuvent être préparés par un procédé qui comprend :

(i) la réaction des composés de formule :

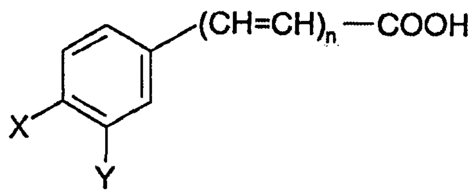

(II)

dans laquelle X et Y ont les mêmes significations que dans la formule (I) ci-dessus, avec le 4-méthyl-3-aminothiophène-2-carboxylate de méthyle de formule :

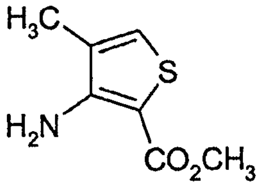

(III)

Pour obtenir les composés de formule :

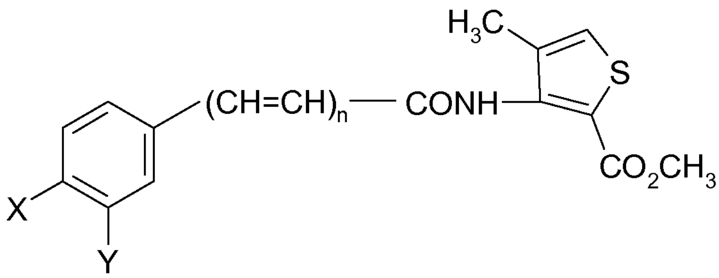

(I')

dans laquelle X, Y et n ont les mêmes significations que dans la formule (I),

(ii) la conversion par hydrolyse du groupe $CO_2CH_3$ présent sur le noyau thiophène des composés (I') obtenus à l'étape (i), en un groupe carboxyle, pour obtenir les dérivés de formule (I) dans laquelle R=OH,
(iii) l'estérification éventuelle du groupe carboxyle des dérivés de formule (I) obtenus à l'étape (ii), par un alcool de formule R'OH où R' a la même signification que ci-dessus, pour obtenir les dérivés de formule (I) dans laquelle R=OR',
(iv) l'amidation éventuelle des dérivés de formule (I) obtenus à l'étape (ii), par une amine de formule $HNR_1R_2$ où $R_1$ et $R_2$ ont les mêmes significations que ci-dessus, pour obtenir les dérivés de formule (I) dans laquelle R= $NR_1R_2$, et
(v) la salification éventuelle des dérivés de formule (I) obtenus à l'étape (ii) avec une base minérale ou organique appropriée.

Quand X dans la formule (II) représente OH, avant la réaction (i) ci-dessus, ce groupe est avantageusement protégé par un groupe protecteur qui peut, par exemple, être un groupe $CH_3CO$, auquel cas ladite réaction (i) est suivie de la déprotection du groupe OH en cause.

Par ailleurs, lors de la réaction (i), le groupe carboxyle du composé de formule (II) est avantageusement mis en oeuvre sous une forme activée, notamment sous la forme d'un anhydride mixte ou d'un chlorure d'acide.

Selon une variante, les dérivés de formule (I) dans laquelle R représente un groupe $NR_1R_2$ où $R_1$ et $R_2$ ont les significations ci-dessus, sont obtenus par conversion respective des dérivés de formule (I) obtenus à l'étape (ii) ci-dessus, en anhydrides symétriques, suivie de la réaction de ces derniers avec un composé de formule $HNR_1R_2$ où $R_1$ et $R_2$ ont les mêmes significations que ci-dessus.

Selon une autre variante, les dérivés de formule (I) dans laquelle R représente un groupe OR' où R' est un groupe dodécane linéaire, peuvent être préparés par réaction des dérivés issus de l'étape (ii) ci-dessus avec le carbonyldiimidazole, pour obtenir des composés de formule :

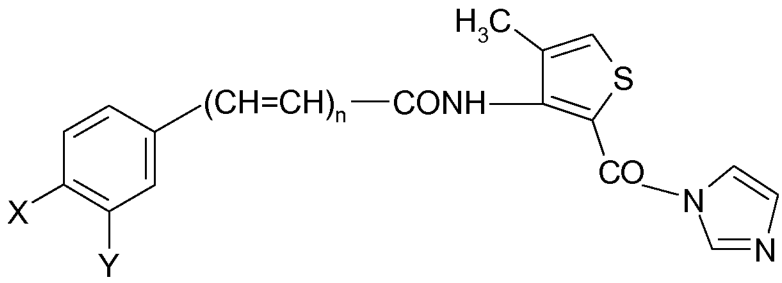

suivie de la condensation de ces derniers avec le D.B.U. (1,8-diazabicyclo[5.4.0]undéc-7-ène).

La préparation d'un certain nombre de dérivés de formule (I) sont rassemblés dans le Tableau 1 ci-dessous et les intermédiaires de synthèse originaux sont rassemblés dans le Tableau 2 ci-dessous. Les composés 3 à 11 du Tableau 1 ne font pas partis de l'invention, ni les intermédiaires du tableau 2 qui ne conduisent pas aux composés (I) de la revendication 1.

Tableau 1

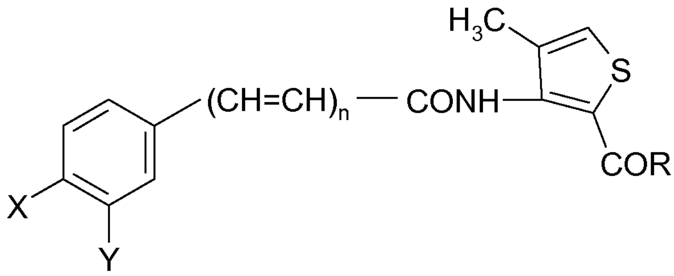

(I)

| Numéro | X | Y | n | R |
|---|---|---|---|---|
| 3 | OH | $OCH_3$ | 1 | $OCH_3$ |
| 4 | OH | $OCH_3$ | 1 | OH |
| 5 | OH | $OCH_3$ | 1 | $OC_2H_5$ |
| 6 | OH | $OCH_3$ | 1 | $OC_3H_7$ |
| 7 | OH | $OCH_3$ | 1 | $OCH(CH_3)_2$ |
| 8 | OH | $OCH_3$ | 1 | $OnC_4H_9$ |
| 8bis | OH | $OCH_3$ | 1 | $O(CH_2)_{11}CH_3$ |
| 10 | OH | $OCH_3$ | 1 | $NH_2$ |
| 11 | OH | $OCH_3$ | 1 | $N(C_2H_5)_2$ |
| 21 | OH | $OCH_3$ | 2 | $OCH_3$ |
| 22 | OH | $OCH_3$ | 2 | OH |
| 23 | OH | $OCH_3$ | 2 | $OC_2H_5$ |
| 24 | OH | $OCH_3$ | 2 | $N(C_2H_5)_2$ |

Tableau 2

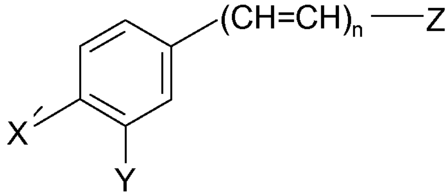

(suite)

| Numéro | X' | Y | n | Z |
|---|---|---|---|---|
| 1 | $CH_3COO$ | $CH_3O$ | 1 | $COO\text{-}COOC_2H_5$ |
| 2 | $CH_3COO$ | $CH_3O$ | 1 | 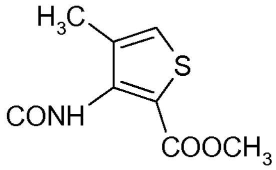 |
| 9 | OH | $CH_3O$ | 1 | 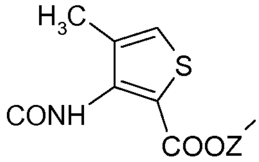 |
| 11 bis | OH | $CH_3O$ | 1 | 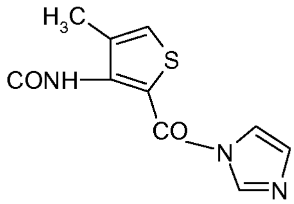 |
| 14 | $t.Bu(Me)_2Si\text{-}O$ | $CH_3O$ | 1 | CHO |
| 15 | $t.Bu(Me)_2Si\text{-}O$ | $CH_3O$ | 2 | $COOC_2H_5$ |
| 15bis | $CH_3COO$ | $CH_3O$ | 2 | $COOC_2H_5$ |
| 16 | OH | $CH_3O$ | 2 | $COOC_2H_5$ |
| 18 | $CH_3COO$ | $CH_3O$ | 2 | COOH |
| 19 | $CH_3COO$ | $CH_3O$ | 2 | $COO\text{-}COOC_2H_5$ |
| 20 | $CH_3COO$ | $CH_3O$ | 2 | 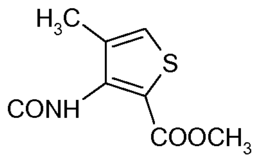 |

Z′ =

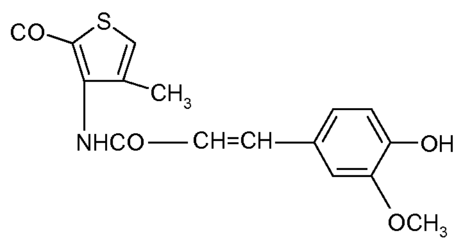

On donnera ci-après un certain nombre d'exemples illustrant la synthèse des dérivés de l'invention ; les numéros apparaissant dans ces exemples correspondent aux composés portant respectivement les mêmes numéros dans les Tableaux 1 et 2 ci-dessus.

Exemple 1 : Acide 4-(acétyloxy)férulique

A température ambiante, 50 g (0,258 mole) d'acide férulique sont mis en solution dans la pyridine. 1,6 g (0,0131 mole) de D.M.A.P est ajouté au milieu réactionnel, puis on ajoute goutte à goutte 27 ml d'anhydride acétique. Après 18 heures d'agitation à température ambiante, la solution est versée dans 1 litre d'eau froide et le précipité formé est essoré, lavé avec 3 fois 500 ml d'eau, séché et recristallisé dans l'éthanol. Poudre blanche : Rdt : 95% ; F : 203°C (litt. 201°C : Ebenezer, W.J., Synth. Commun. 1991, 21(3), 351-8).

Exemple 2 :

Acétate de 4-{(1E)-3-[(éthoxycarbonyl)oxy]-3-oxoprop-1-èn-1-yl}-2-méthoxyphényle (1).

A 0°C, 20 g (0,0847 mole) d'acide 4-(acétyloxy) férulique sont dissous dans 200 ml d'acétonitrile. 11,9 ml (0,0847 mole) de triéthylamine sont ajoutés goutte à goutte puis la solution est agitée pendant une heure à cette température. 9,2 ml (0,0847 mole) de chloroformiate d'éthyle sont ensuite additionnés goutte à goutte. Après 1 heure d'agitation à 0°C, le chlorhydrate de triéthylammonium formé est filtré, lavé avec 30 ml d'acétonitrile, puis le filtrat est concentré sous pression réduite. L'huile résiduelle est additionnée de 200 ml d'eau froide puis la solution est extraite par 200 ml d'acétate d'éthyle.

La phase organique est séchée sur sulfate de magnésium, filtrée puis évaporée sous pression réduite. On obtient sans autre purification 24 g (Rdt=92%) du composé anhydride sous la forme de cristaux blancs.
F=90°C.
IR (KBr)ν($cm^{-1}$) : 1800, 1762, 1726 (C=0).
RMN$^1$H (DMSO-d6) : 1,26 (t,3H,0$CH_2CH_3$) ; 2,23 (s,3H, $OCOCH_3$) ; 3,79 (S, 3H, $OCH_3$) ; 4,28 (q, 2H,0$CH_2$$CH_3$) ; 6, 73 (d, 1H, $H_2$) ; 7,13 (d, 1H, $H_{5'}$) ; 7,33 (d, 1H, $H_{6'}$) ; 7,52 (s, 1H, $H_{2'}$) ; 7, 82 (d, 1H, $H_3$).
Masse (ESI) : $[M+H]^+$ 309.
$C_{15}H_{16}O_7$ : 308,29.

Exemple 3 :

3-({(2E)-3-[4-(acétyloxy)-3-méthoxyphényl] prop-2-ènoyl} amino)-4-méthylthiophène-2-carboxylate de méthyle (2)

On agite dans un ballon un mélange intime de 27 g (0,0876 mole) d'anhydride mixte (1) et de 15 g (0,0876 mole) de M.A.T.C.M (méthyl-4-amino-3-thiophène-2-carboxylate de méthyle) sans solvant puis on chauffe à 130°C au bain d'huile en maintenant une agitation douce pendant 40 minutes. On laisse refroidir vers 80-90°C et on ajoute 80 ml d'acétonitrile, l'huile résiduelle passe en solution, on refroidit vers 25°C, on ajoute 300 ml d'éther éthylique et on poursuit l'agitation pendant 8 heures. Le précipité blanc est essoré, lavé à l'éther éthylique et séché. On obtient sans autre purification 27g (Rdt=79%) du dérivé attendu.
F : 138°C.
IR (KBr)ν($cm^{-1}$) : 1765, 1700, 1664 (C=0).
RMN$^1$H ($CDCl_3$) : 2,28 (s, 3H, $CH_3$ (thiophène); 2,34 (s, 3H, $COCH_3$) ; 3,87 (s, 3H, $CO_2CH_3$) ; 3,89 (s, 3H, $OCH_3$) ; 6,61 (d, $J_{2-3}$=15,6Hz, 1H, $H_2$) ; 7,06-7,18 (m, 4H, $H_{2'}$, $H_{5'}$, $H_{6'}$ et H thiophène); 7,69 (d, $J_{3-2}$=15,6Hz, 1H, $H_3$) ; 9, 12 (s, 1H, NH).
Masse (ESI): $[M+H]^+$ 390.
$C_{19}H_{19}NO_6S$ : 389,43.

Exemple 4 :

3-{[(2E)-3-(4-hydroxy-3-méthoxyphényl)prop-2-ènoyl] amino}-4-méthylthiophène-2-carboxylate de méthyle (3)

On agite à température ambiante 4g (0,0103 mole) de (2) dans un mélange eau-tétrahydrofurane 30ml/30ml en présence de 2,84g (0,0205 mole) de carbonate de potassium puis la solution est chauffée à 45°C pendant 3 heures. Le THF est évaporé, la phase aqueuse est reprise par 40 ml d'eau froide puis acidifiée sous agitation à pH=1 avec de l'acide chlorhydrique (0,5N). Le précipité blanc est essoré, lavé à l'eau, séché et recristallisé dans un mélange méthanol/eau (3/2). Cristaux blancs, F=176°C, poids=2,50 g, Rdt=70%.
IR (KBr)ν($cm^{-1}$)=3300(NH) ; 3139 (0H); 1706 et 1651 (C=0).
RMN$^1$H ($CDCl_3$) : 2,27 (s, 3H, $CH_3$ thiophène); 3,87 (s, 3H, $CO_2CH_3$) ; 3,95 (s, 3H, $OCH_3$) ; 5,88 (s, 1H, 0H); 6, 51 (d, 1H, $H_2$) ; 6, 93 (d, 1H, $H_{5'}$) ; 7, 07 (d, $J_{2'-6'}$= 2Hz, 1H, $H_2$) ; 7,11 (dd, 1H, $H_{6'}$) ; 7,17 (s, 1H, H thiophène); 7, 66 (d, 1H, $H_3$) ; 9,09 (s, 1H, NH).
Masse (ESI) : $[M+H]^+$ 348.
$C_{17}H_{17}NO_5S$ : 347,39.

Exemple 5 :

Acide 3-{[(2E)-3-(4-hydroxy-3-méthoxyphényl)prop-2-ènoyl]amino}-4-méthylthiophène-2-carboxylique (4)

On agite 2 heures à 50°C dans 120 ml d'un mélange eau/tétrahydrofurane (1/1), 20g (0,051 mole) de dérivé (2) et 2,7 g (0,0117 mole)de LiOH. Le mélange de réaction est refroidi à 5°C puis acidifié sous agitation forte par une

solution aqueuse d'acide chlorhydrique 1N jusqu'à pH=1. Après 2 heures d'agitation, le précipité blanc formé est essoré, lavé à l'eau froide puis avec 50 ml d'acétonitrile et séché sous vide. On obtient sans autre purification 15,4 g (Rdt : 90%) du composé attendu sous la forme d'une poudre blanche.
F: 255°C (déc).
IR(KBr)ν($cm^{-1}$) : 3300(NH, OH) ; 1661, 1625 (C=0) ; 1594; 1565; 1490; 135; 1167; 1036; 970 et 566.
RMN$^1$H(DMSO-$d_6$) : 2, 12 (s, 3H, $CH_3$ thiophène) ; 3,81 (s, 3H, $0CH_3$) ; 6,69 (d, $J_{2-3}$=15,6Hz, 1H, $H_2$). 6,82 (d, $J_{5'-6'}$=7, 8Hz, 1H, $H_{5'}$) ; 6, 96 (s, 1H, $H_{2'}$); 7, 05 (d, $J_{5'-6'}$=7,8Hz, 1H, $H_{6'}$) ; 7,26 (s, 1H, H thiophène); 7,41 (d, $J_{2-3}$=15,6Hz, 1H, $H_3$) ; 10, 95 (s, 1H, NH).
Masse (ESI): $[M+H]^+$ 334. $C_{16}H_{15}NO_5S$ : 333, 37.

Exemple 6 :

3-{[(2E)-3-(4-hydroxy-3-méthoxyphényl)prop-2-ènoyl] amino}-4-méthylthiophène-2-carboxylate d'éthyle (5)

On porte au reflux 4 heures dans 100 ml d'éthanol absolu en présence de 0,3 ml d'acide sulfurique, 2 g (0,0060 mole) de dérivé (4). Après refroidissement, la solution est concentrée sous vide, le résidu est repris par 60 ml d'eau puis extrait par 120 ml d'acétate d'éthyle. La phase organique est séchée puis évaporée sous pression réduite, l'huile résiduelle cristallise par addition d'un mélange éther-acétonitrile (3-1). Poudre blanche, F : 201°C, poids : 1,6 g, Rdt : 74%.
IR(KBr)ν($cm^{-1}$) : 3340 (NH, OH); 1700; 1665 (C=0).
RMN$^1$H(DMSO-$d_6$) : 1,07 (t, 3H, $CO_2CH_2CH_3$) ; 2,04 (s, 3H, $CH_3$ thiophène); 3,81 (s, 3H, $OCH_3$) ; 3,36 (9, 2H, $CO_2CH_2CH_3$) ; 6, 74 (d, 1H, $H_2$) 6, 81 (d, 1H, $H_{5'}$) ; 7,06 (d, 1H, $H_{6'}$) ; 7,20 (s, 1H, $H_{2'}$) ; 7, 46 (m, 2H, $H_3$ et H thiophène); 9, 51 (s, 1, OH); 9,61 (s, 1H, NH).
Masse (ESI): $[M+H]^+$ 362. $C_{18}H_{19}NO_5S$ : 361,34.

Exemple 7 :

3-{[(2E)-3-(4-hydroxy-3-méthoxyphényl)prop-2-ènoyl] amino}-4-méthylthiophène-2-carboxylate de propyle (6)

On utilise le mode expérimentale de l'exemple 6. La réaction est réalisée en utilisant 2 g (0,0060 mole) du dérivé (4), 0, 3 ml d'acide sulfurique et 100 ml de propanol. Poudre blanche, F: 220°C, poids: 1,5 g, Rdt: 67%.
IR(KBr)ν($cm^{-1}$) : 3320 (NH, OH) ; 1705; 1650 (C=0).
RMN$^1$H(DMSO-$d_6$) : 1, 06 (t, 3H, $CO_2(CH_2)_2$-$CH_3$) ; 2, 06 (m, 4H, $CH_3$ thiophène, $CH_2$) ; 3, 37 (q, 2H, $CO_2CH_2CH_2CH_3$) ; 3,81 (s, 3H, $OCH_3$) ; 6, 74 (d,$J_{2-3}$=15,6Hz, $H_2$) ; 6, 81 (d,$J_{5'-6'}$=8Hz, 1H, $H_{5'}$) ; 7,06 (d,$J_{6'-5'}$=8Hz, 1H, $H_{6'}$) ; 7,20 (s, 1H, $H_{2'}$) ; 7, 46 (m, 2H, $H_3$ et H thiophène); 9,52 (s, 1H, OH); 9,62 (s, 1H, NH).
Masse (ESI): $[M+H]^+$ 376. $C_{19}H_{21}NO_5S$: 375, 37.

Exemple 8 :

3-{[(2E)-3-(4-hydroxy-3-méthoxyphényl)prop-2-ènoyl] amino}-4-méthylthiophène-2-carboxylate d'isopropyle (7)

On utilise le mode expérimentale de l'exemple 6. La réaction est réalisée en utilisant 2 g (0,0060 mole) du dérivé (4), 0, 3 ml d'acide sulfurique et 100 ml d'isopropanol. Poudre blanche, F : 218°C, poids: 0,8 g, Rdt : 36%.
IR(KBr)ν($cm^{-1}$) : 3340 (NH, OH) ; 1700; 1660 (C=0).
RMN$^1$H(DMSO-$d_6$): 1,20 (m, 6H, $CH(CH_3)_2$) ; 2,05 (s, 3H, $CH_3$ thiophène); 3, 81 (s, 3H, $OCH_3$) ; 4, 36 (m, 1H, $CH(CH_3)_2$) ; 6,74 (d, $J_{2-3}$=15, 6Hz, 1H, $H_2$) ; 6,80 (d, $J_{H5'H6'}$=7, 8Hz, 1H, $H_{5'}$); 7,20 (s, 1H, $H_{2'}$) ; 7, 45 (s, 1H, H thiophène); 7, 48 (d, 1H, $H_3$) ; 9,51 (s, 1H, OH); 9,61 (s, 1H, NH).
Masse (ESI): $[M+H]^+$ 376. $C_{19}H_{21}NO_5S$: 375, 37.

Exemple 9 :

3-{[(2E)-3-(4-hydroxy-3-méthoxyphényl)prop-2-ènoyl] amino}-4-méthylthiophène-2-carboxylate de butyle (8)

On utilise le mode expérimentale de l'exemple 6. La réaction est réalisée en utilisant 2 g (0,0060 mole) du dérivé (4), 0,3 ml d'acide sulfurique et 70 ml de 1-butanol. Poudre blanche, F : 196°C, poids: 1 g, Rdt : 43%.
IR(KBr)ν($cm^{-1}$) : 3320 (NH, OH); 1710 ; 1660 (C=0).
RMN$^1$H(DMSO-$d_6$) : 0,86 (t, 3H, $CH_3$) ; 1,34 (m, 2H, $CH_2$) ; 1,60 (m, 2H, $CH_2$) ; 2,05 (s, 3H, $CH_3$ thiophène); 3,50 (m, 2H, $CH_2$) ; 3, 80 (s, 3H, $OCH_3$) ; 6,74 (d, 1H, $H_2$) ; 6,80 (d, 1H, $H_{5'}$) ; 7,06 (d, 1H, $H_{6'}$) ; 7,20 (s, 1H, $H_{2'}$) ; 7,46 (m, 2H, $H_3$ et H thiophène); 9,52 (s, 1H, OH); 9,62 (s, 1H, NH).

Masse (ESI): $[M+H]^+$ 390. $C_{20}H_{23}NO_5S$ : 389, 39.

Exemple 10 :

3-{[(2E)-3-(4-hydroxy-3-méthoxyphényl)prop-2-ènoyl] amino}-4-méthylthiophène-2-carboxylate de dodécyle (8 bis)

On porte au reflux sous azote et sous agitation, 70 ml de tétrahydrofurane anhydre et 2 g (0,0522 mole) de dérivé imidazole (11bis) préparé à l'Exemple 14, en présence de 0, 80g (0,0522 mole) de D.B.U (1,8-diazabicyclo[5.4.0]undéc-7-ène), pendant 20 heures. Après refroidissement, la solution est concentrée sous vide, le résidu est repris dans 80 ml d'eau froide, agitée 30 minutes et filtré sur fritté. Le précipité obtenu est séché et recristallisé dans l'acétonitrile. Poudre rosée, F : 202°C (déc.), poids: 1,4 g, Rdt : 54%. $C_{28}H_{39}NO_5S$ : 501,50.
IR(KBr)$\nu$($cm^{-1}$) : 3400 (OH) ; 3230 (NH) ; 1700, 1660 (C=0).
RMN$^1$H(DMSO-$d_6$) : 0, 83 (t, 3H, $CH_3$) ; 1,22 (m, 18H, $(CH_2)_9$; 1, 70 (m, 2H, $CH_2$) ; 2,28 (s, 3H, $CH_3$ thiophène) ; 3, 81 (s, 3H, $OCH_3$) ; 4, 34 (t, 2H, $OCH_2$) ; 6,61 (s, 1H, OH) ; 7,06, 7,11 (dd, 2H, $H_2$, $H_{5'}$) ; 7, 30 (d,$J_{2'-6'}$=2Hz, 1H, $H_{2'}$) ; 7,58 (d,$J_{6'-5'}$=8Hz, 1H, $H_{6'}$) ; 7,68 (d,$J_{3-2}$=15,6Hz, 1H, $H_3$) ; 7,99 (s, 1H, H thiophène); 8,24 (s, 1H, NH).
Masse (ESI) : $[M+H]^+$ 502.

Exemple 11:

Anhydryde 3-{[(2E)-3-(4-hydroxy-3-méthoxyphényl)prop-2-énoyl]amino}-4-méthylthiophène-2-carboxylique (9)

On agite 0°C 3g (0,0090 mole) de dérivé (4) puis on additionne goutte à goutte 1,26 ml (0,0124 mole) de triéthylamine et on poursuit l'agitation pendant 30 minutes ; on ajoute ensuite 0,86 ml (0,0079 mole) de chloroformiate d'éthyle goutte à goutte et on laisse agiter 30 minutes. Le précipité formé est filtré sur fritté (chlohydrate de triéthylamine) et la solution est concentrée sous pression réduite. Le résidu est repris par 70 ml d'eau froide et extrait par 120 ml d'acétate d'éthyle. La phase organique est décantée, séchée sur sulfate de magnésium et concentrée sous vide. L'huile résiduelle cristallise dans 20 ml d'acétonitrile, on filtre sur fritté et on lave avec 10 ml d'acétonitrile. Après séchage, on obtient sans autre purification 3,10g (Rdt=53%) du dérivé anhydride symétrique (9). Poudre jaune, F= 202°C.
IR(KBr)$\nu$($cm^{-1}$) 3432 (OH), 1800, 1651 (C=0).
RMN$^1$H (DMSO-$d_6$) : 2,30 (s, 3H, $CH_3$ thiophène); 3,87 (s,3H, $0CH_3$) ; 6,81 (d, $J_{5'-6'}$= 7,8Hz, 1H, $H_{5'}$) ; 6, 91 (d, $J_{2-3}$= 15, 6 Hz, 1H, $H_2$) ; 7,20 (dd, $J_{5'-6'}$= 7, 8Hz, $J_{6'-2'}$ = 2 Hz, 1H, $H_{6'}$) ; 7,43 (s, 1H, $H_{2'}$) ; 7,71 (d,$J_{3-2}$= 15, 6 Hz, 1H, $H_3$) ; 8,03 (s, 1H, H thiophène); 9,80 (s, 1H, 0H).
$C_{32}$ $H_{28}$ $N_2$ $0_9$ $S_2$ = 648, 56

Exemple 12 :

3-{[(2E)-3-(4-hydroxy-3-méthoxyphényl)prop-2-énoyl]amino}-4- méthylthiophène-2-carboxamide (10)

On chauffe à 80°C sous agitation 70ml de dioxane saturé d'ammoniac gaz et 1g (0,00154 mole) de dérivé anhydride symétrique (9) pendant 4 heures. Après refroidissement, le précipité formé est essoré, lavé avec 15ml d'acétonitrile et 10 ml d'éther éthylique. Poudre jaune claire, F = 202°C, poids : 0,2g, Rdt : 39%.
IR(KBr)$\nu$(cm-1) : 3432, 3280, 3260 (OH,NH,NH2), 1670, 1660 (C=0).
RMN$^1$H (DMSO-$d_6$) : 2,31 (s, 3H, $CH_3$ thiophène); 3,82 (s, 2H, $OCH_3$) ; 5, 10 (s, 2H, $NH_{2'}$) ; 6,64 (d, $J_{2-3}$ = 15,62 Hz, 1H, $H_2$) : 6, 81 (d, $J_{5'-6'}$= 8, 79Hz, 1H, $H_{5'}$) ; 7, 03 (d, 1H, $H_{6'}$) ; 7, 16 (s, 1H, $H_{2'}$) ; 7,23 (s, 1H, H thiophène); 7,43 (d, $J_{3-2}$= 15, 62Hz, 1H, $H_3$) ; 9,48 (s, 1H, OH); 9, 57 (s, 1H, NH).
$C_{16}$ $H_{16}$ $N_2$ $0_4$ S : 332, 30.

Exemple 13 :

N,N'-diéthyl-3-{[(2E)-3-(4-hydroxyphényl)-prop-2-énoyl]amino}-4-méthylthiophène-2- carboxamide (11)

On porte au reflux 3 heures dans 30 ml d'acétonitrile 1g (0,00154 mole) d'anhydride symétrique (9) et 0,56g (0,0077 mole) de diéthylamine. Après refroidissement, le précipité jaune est essoré, lavé à l'éther éthylique, séché et recristallisé dans l'acétonitrile. Poudre jaune foncé. F: 172°C, poids: 0,3 g, Rdt: 50%.
IR(KBr)$\nu$(cm-1) : 3420, 3280 (OH, NH); 1665, 1660 (C=0).
RMN$^1$H (DMSO-$d_6$) : 1,06 (t, 6H, $(CH_2CH_3)_2$) ; 2, 05 (s, 3H, $CH_3$ thiophène) ; 3,32 (q, 4H, $(CH_2CH_3)_2$) ; 3, 80 (s, 3H, $OCH_3$); 6,64 (d, 1H, $H_2$) ; 6, 78 (d, 1H, $H_{5'}$) ; 7,03 (d, 1H, $H_{6'}$) ; 7,16 (s, 1H, $H_{2'}$) ; 7,24 (s, 1H, H thiophène ; 7,43 (d, 1H, $H_3$) ; 9,57 (s, 1H, OH); 9,59 (s, 1H, NH).

$C_{20}H_{24}N_20_4S$: 388,41.

Exemple 14:

(2E)-3-(4-hydroxy-3-méthoxyphényl)-N-[2-(imidazole-1-carbonyl)-4-méthyl-thiophèn-3-yl]-acrylamide (11 bis)

On porte au reflux 3 heures dans 70ml de tétrahydrofurane anhydre 3g (0,009 mole) de dérivé acide (4) en présence de 2,91g (0,0180 mole) de carbonyldiimidazole. Après refroidissement de la solution, le précipité jaune formé est essoré, lavé avec 30 ml d'éther éthylique, séché et directement engagé pour l'obtention du carboxylate de dodécyle (8bis). Poudre jaune, F=238°C, poids = 2,9 g, Rdt = 84%.
$C_{19}H_{17}N_30_4S$ = 383, 35
IR (KBr)ν(cm$^{-1}$) : 3400 (OH); 3380 (NH) ; 1630 (C=0).
RMN$_1$H (DMS0-$d_6$) : 2,33 (s, 3H, $CH_3$ thiophène); 3,88 (s, 3H, $OCH_3$) ; 7,16 (m, 2H, $H_2$-$H_{2'}$) ; 7,48 (m, 2H, $H_{5'}$, 1H imidazole); 7,75 (m, 2H, $H_{6'}$, 1H imidazole); 7,85 (d, $J_{3-2}$=15,6 $H_z$, $H_3$) ; 8,03 (s, 1H, H thiophène); 8,43 (s, 1H, H imidazole); 9,57 (s, 2H, OH, NH).
Masse (ESI) : $[M+H]^+$ : 384

Exemple 15 :

(2E)-3-[4-[[tert-butyldiméthylsilyl]oxy]-3-méthoxyphényl] prop-2-énoate de méthyle (12)

24,5g (0,360 mole) d'imidazole sont ajoutés à une solution de 30 g (0,144 mole) de férulate de méthyle et de 26,1g de chlorure de tert-butyldiméthylsilyle (0,173 mole) dans le diméthylformamide. La solution est agitée à température ambiante 2 heures. La solution organique est additionnée de 120 ml d'eau puis extraite à l'éther éthylique, la phase étherée est lavée par une solution de chlorure de sodium, puis à l'eau et séchée sur $MgS0_4$. Le produit brut obtenu est purifié par chromatographie sur colonne de gel de silice (éluant : cyclohexane / acétate d'éthyle : 9/1) ; poudre blanche, F : 65°C, poids : 45,8g, Rdt : 99% (Littérature : 64°C-J. Mol. Catal.B : Enzym. 2006, 40, (1-2), 8-15).

Exemple 16 :

(2E)-3-[4-[[tert-butyldiméthylsilyl]oxy]-3-méthoxyphényl]prop-2-èn-1-ol (13)

A -10°C sous argon, 340 ml de DIBAL-H (solution 1M dans le tétrahydrofurane) sont ajoutés goutte à goutte en 30 minutes à une solution du composé (12) [21, 9g (0,0679 mole) dans 300 ml de THF anhydre]. La solution est ensuite agitée pendant 6 heures à température ambiante. A 0°C, 100 ml d'eau sont ajoutés goutte à goutte et une solution aqueuse d'HCl 2N est ajoutée jusqu'à pH=4-5. Après décantation, la phase aqueuse est extraite par 2 fois 100ml d'éther éthylique. Le produit brut obtenu est purifié par chromatographie sur colonne de gel de silice (éluant : cyclohexane/acétate d'éthyle : 9/1). On obtient 18,6 g (Rdt= 93%) du composé (13) sous la forme d'une huile incolore (Littérature: 64°C-*J*. Mol. Catal.B: Enzym. 2006, 40, (1-2), 8-15).

Exemple 17 :

(2E)-3-[4-[[ter-butyldiméthylsilyl]oxy]-3-méthoxyphény]acrylaldéhyde (14)

A température ambiante, 70,4 g (0,810 mole) de $MnO_2$ sont ajoutés à une solution du composé (13) (15,9g, 0,054 mole) dans 200 ml de dichlorométhane. Le milieu réactionnel est agité pendant 24 heures. Le mélange est filtré sur Célite et rincé plusieurs fois à l'acétate d'éthyle. Après évaporation, on obtient 14,1 g (Rdt : 89%) du composé (14) sous la forme d'une poudre blanche. $C_{16}H_{24}O_3Si$ : 292,45. F: 79°C.
IR(KBr)ν (cm-1) : 1670 (C=O aldéhyde) ; 1621; 1596; 1511; 1464; 1424; 1283; 1164; 1129; 1034; 984; 906; 837; 783.
RMN$^1$H ($CDCl_3$) : 0,18 (s, 6H, $(CH_3)_2$-Si) ; 1,00 (s, 9H, $(CH_3)_3$C-Si) ; 3,85 (s, 3H, -OMe) ; 6,60 (dd, $J_{2-3}$ = 15, 9 Hz et $J_{2-Hald}$ = 7, 8 Hz, 1H, H2); 6,88 (d, $J_{5'-6'}$ = 8, 0 Hz, 1H, $H_{5'}$) ; 7, 05-7, 09 (m, 2H, $H_2$, et $H_{6'}$) ; 7,40 (d, $J_{3-2}$ = 15, 9 Hz, 1H, $H_3$) ; 9,66 (d, $J_{Hald-2}$ = 7,8 Hz, 1H, $H_{ald}$).
RMN$^{13}$C ($CDCl_3$) : -4,5 (($\underline{C}H_3)_2$-Si) ; 18,4 (($CH_3)_3\underline{C}$-Si) ; 25,6 (($\underline{C}H_3)_3\underline{C}$-Si) ; 55, 3 (-OMe); 111,0; 121,2; 123,1; 126,7; 127,9; 148,6; 151,4; 153,1; 193,7 (C1).
Masse (ESI) : $[M+H]^+$ : 293.

Exemple 18 :

(2E,4E)-5[4-[[tert-butyldiméthylsily]oxy]-3-méthoxyphényl]penta-2,4-diènoate d'éthyle (15)

A 0°C, 2,1 g (0,088 mole) d'hydrure de sodium sont ajoutés par petites portions à une solution de 11 g (0,490 mole) de (diéthoxyphosphoryl) acétate d'éthyle et 13 g (0,0445 mole) du dérivé acrylaldéhyde (14) dans 70 ml de DMF. Après 1 heure d'agitation à température ambiante, de l'eau glacée est ajoutée au milieu réactionnel. La phase aqueuse est extraite à l'éther éthylique. Le produit brut est purifié par chromatographie sur colonne de gel de silice (éludant : cyclohexane/acétate d'éthyle : 95/5). On obtient 15,60 g (Rdt : 97%) du composé (15) sous la forme d'un solide incolore. $C_{20}$ $H_{30}$ $O_4$ Si: 362, 55 ; F: 48°C.
IR(KBr)ν($cm^{-1}$) : 1710 (C=O ester); 1625; 1594; 1511; 1464; 1418; 1367; 1313; 1285; 1264; 1174; 1132; 1038; 998; 905; 841; 806; 783; 703.
RMN$^1$H ($CDCl_3$) : 0,16 (s, 6H, $(CH_3)_2$-Si) ; 0, 99 (s, 9H, $(CH_3)_3$C-Si ; 1, 31 (t, *J*=7,1 Hz, 3H, -$OCH_2CH_3$) ; 3,84 (s, 3H,-OMe) ; 4,22 (q, *J* = 7, 1 Hz, 2H, -$OCH_2CH_3$) ; 5,94 (d, $J_{2-3}$ = 15, 4 Hz, 1H, $H_2$) ; 6,73 (dd, $J_{4-5}$ = 15,6 Hz et $J_{4-3}$ = 10,6 Hz, 1H, $H_4$) ; 6, 81-6, 85 (m, 2H, $H_5$ et $H_{5'}$) ; 6,93-6,96 (m, 2H, $H_{2'}$ et $H_{6'}$) ; 7,43 (dd, $J_{3-2}$ = 15, 4 HZ et $J_{3-4}$ = 10,6 Hz, 1H, $H_3$).
RMN$^{13}$C ($CDCl_3$) : -4, 6 ($(CH_3)_2$-Si) ; 14, 3 (-$OCH_2CH_3$) ; 18,5 ($(CH_3)_3C$-Si) ; 25,7 ($(CH_3)_3C$-Si) ; 55,5 (-OMe); 60,2 (-$OCH_2CH_3$); 110,2; 120,2; 121,0; 121,1; 124,4; 130,0; 140,5; 144, 9; 146, 4; 151,2; 167, 3 (C1).
Masse (IE, 70 eV) : *m/z* (%) : 362 (6) [$M^+$] ; 305 (24) [$M^+$-*t*-Bu]; 290 (6) ; 245 (5) ; 217 (29) ; 121 (29); 119 (37). Masse (ESI) : $[M+H]^+$ 363.
Analyse élémentaire : théorique C 66,26%, H 8,34% ; expérimentale C 66,47%, H 8,69%.

Exemple 19 :

(2E,4E)-5[4-acétyloxy-3-méthoxyphényl]penta-2,4-diènoate d'éthyle (15 bis)

A 0°C, 0,158 g (0,0066 mole) d'hydrure de sodium est ajouté par petites fractions à une solution de 0,988 g (0,0039 mole) de (2E)-4-(diéthoxyphosphoryl)but-2-ènoate d'éthyle et 0,639 g (0,00329 mole) d'acétate de 4-formyl-2-méthoxy phényle dans 20 ml de DMF. Après 1 heure d'agitation à température ambiante, de l'eau glacée est ajoutée au milieu réactionnel. La phase aqueuse est extraite à l'éther éthylique. Le produit brut est purifié par chromatographie sur colonne de gel de silice (éluant: cyclohexane/acétate d'éthyle : 9/1). On obtient 0,716 g (Rdt= 75%) du composé (15 bis) sous forme de cristaux blancs. C16 H18 O5: 290,32.
F: 81°C.
IR(KBr)ν($cm^{-1}$): 1762 (C=O acétoxyle); 1704 (C=O ester C1); 1629; 1513; 1370; 1318; 1241; 1217; 1197; 1162; 1119 ; 1032; 1002; 850.
RMN$^1$H ($CDCl_3$): 1,32 (t, J= 7, 1 Hz, 3H, -$OCH_2CH_3$); 2,32 (s, 3H, -$OCOCH_3$); 3,87 (s, 3H, -OMe) ; 4,23 (q, J= 7, 1 Hz, 2H, -$OCH_2CH_3$) ; 5,99 (d, $J_{2-3}$= 15,3 Hz, 1H, $H_2$) ; 6,80 (dd, $J_{4-5}$= 15, 4 Hz et $J_{4-3}$= 9, 7 Hz, 1H, $H_4$); 6,86 (d, $J_{5-4}$= 15,4 Hz, 1H, $H_5$) ; 6,99-7,07 (m, 3H, $H_{2'}$, $H_{5'}$ et $H_{6'}$); 7,43 (dd, $J_{3-2}$= 15,3 Hz et $J_{3-4}$= 9,7 Hz, 1H, $H_3$).
RMN$^{13}$C ($CDCl_3$) : 14,5 (-$OCH_2CH_3$) ; 20, 8 (-$OCOCH_3$) ; 56,1 (-OMe) ; 60,5 (-$OCH_2CH_3$) ; 110,7; 120,3; 121,7; 123,3; 126,6; 135,2; 139,7; 140,6; 144,4; 151,5; 167,1 (C1) ; 169, 1 (-$OCOCH_3$).
Masse (ESI) : $[M+H]^+$ 291.

Exemple 20 :

(2E,4E)-5-[4-hydroxy-3-méthoxyphényl]penta-2,4-diénoate d'éthyle (16)

A O°C et sous argon, 15g (0,0413 mole) du dérivé (15) sont mis en solution dans 100 ml de THF antydre. 8 ml d'acide acétique glacial sont ensuite ajoutés. Puis 82,7 ml de TBAF (solution 1M dans le THF) sont additionnés goutte à goutte. Après 1 heure, le THF est évaporé sous pression réduite et le pH est ensuite ajusté à 4-5 par une solution aqueuse d'HCl 0,5 N. La phase aqueuse est extraite par de l'éther diéthylique ; on obtient 9,6 g (Rdt = 93%) du composé (16) sous la forme d'un solide jaune. $C_{14}H_{16}O_4$ : 248,28.
F: 90°C.
IR(KBr)ν($cm^{-1}$): 3430 (O-H); 1684 (C=O ester); 1624; 1587; 1512; 1336; 1259; 1208; 1185; 1137; 1031; 1017; 1004; 864; 806.
RMN$^1$H ($CDCl_3$): 1,31 (t, J=7, 1 Hz, 3H, -$OCH_2CH_3$); 3, 93 (s,3H,-OMe); 4,22 (q, J=7,1 Hz, 2H, -$OCH_2CH_3$); 5, 78, (s large, 1H, OH); 5, 94 (d, $J_{2-3}$= 15,2 Hz, 1H, $H_2$), 6,72 (dd, $J_{4-5}$= 15,3 Hz et $J_{4-3}$= 10, 7 Hz, 1H, $H_4$); 6,82 (d, $J_{5-4}$ = 15,3 Hz, 1H, $H_5$) ; 6, 89 (d, $J_{5'-6'}$= 8, 1 Hz, 1H, $H_{5'}$) ; 6, 97 (d, $J_{2'-6'}$= 1,7 Hz, 1H, $H_{2'}$) ; 6, 99 (dd, $J_{6'-5'}$= 8, 1 Hz et $J_{6'-2'}$= 1, 7 Hz, 1H, $H_{6'}$); 7,43 (dd, $J_{3-2}$ = 15,2 Hz et $J_{3-4}$ = 10,7 Hz, 1H, $H_3$).

RMN$^{13}$C ($CDCl_3$) : 14,3 ($-OCH_2CH_3$) ; 56, 0 (-OMe); 60, 3 ($-OCH_2CH_3$) ; 108,7; 114,7; 120, 1; 121,7; 124, 1; 128,7; 140,5; 144, 9; 146, 8; 146, 9; 167, 3 (C1).
Masse (ESI): $[M-H]^+$ 247.

Analyse élémentaire: théorique C 67,73%, H 6,50%; expérimentale C 67,39%, H 6,76%.

Exemple 21 :

Acide (2E,4E)-5-[4-hydroxy-3-méthoxyphényl]penta-2,4-diénoique (17)

8g (0,0322 mole) d'ester (15) sont dissous dans 50 ml d'éthanol en présence de 75 ml d'une solution aqueuse d'hydroxyde de potassium à 10%. Après 4 heures de reflux, l'alcool est évaporé et la solution est acidifiée à froid par une solution aqueuse d'HCl 2N. On obtient 6,4g (Rdt : 90%) du dérivé (17) sous la forme d'un solide orangé. $C_{12}H_{12}O_4$ : 220,23.
F: 199°C. (litt. 190-192°C)
IR(KBr)ν(cm$^{-1}$) : 3525 (O-H phénol); 3457 (O-H acide); 1681 (C=O acide); 1587; 1514; 1429; 1336; 1311; 1267; 1209; 1164; 1125; 1022; 994; 926; 853; 819; 761; 699; 606 ; 549.
RMN$^1$H (DMSO-d6): 3,80 (s, 3H, -OMe); 5,89 (d, $J_{2-3}$ = 15,0 Hz, 1H, $H_2$) ; 6,76 (d,$J_{6'-5'}$ = 8,1 Hz, 1H, $H_{6'}$) ; 6,92-6,98 (m, 3H, $H_4$, $H_{5'}$ et $H_5$) ; 7,15 (s, 1H, $H_{2'}$) ; 7,30 (ddd, $J_{3-2}$= 15, 1 Hz, $J_{3-4}$ = 6,9 Hz et $J_{3-5}$ = 3, 0 Hz, 1H, $H_3$); 9,76 (s large, 1H, OH phénol); 12,1 (s large, 1H, OH acide).
RMN$^{13}$C (DMSO-$d_6$): 56,0 (-OMe); 110,6; 115,9; 120,4; 121,9; 124,0; 128,1; 141,4; 145,7; 148,2 (2C); 168,3 (C1).
Masse (IE, 70 eV): *m/z* (%): 220 (52) [$M^+$] ; 175 (100) [$M^+ - CO_2H$]; 160 (28) ; 143 (19) ; 131 (20) ; 115 (42).
Masse (ESI) : $[M-H]^+$ 219.

Exemple 22 :

Acide (2E,4E)-5-[4-acétyloxy-3-méthoxyphényl]penta-2,4-diènoique (18)

On agite à température ambiante dans 70 ml de pyridine, 20 g (0,0908 mole) du dérivé (17) en présence de 9,5 ml d'anhydride acétique et de 0,6 g de DMAP (0,049 mole). Après 24 heures d'agitation à température ordinaire, la solution est concentrée sous pression réduite, le résidu est repris par 150 ml d'une solution aqueuse d'HCl 0,5 N puis extraite par 2 fois 200 ml d'acétate d'éthyle. On obtient sans autre purification 21,9 9 g (Rdt = 92 %) du composé (18) sous la forme d'un solide beige. $C_{14} H_{14} O_5$ : 262, 26.
F: 210°C.
IR(KBr)ν(cm$^{-1}$) : 3436 (O-H) ; 1766 (C=O ester); 1674 (C=O acide); 1612; 1511; 1423; 1313; 1268; 1208; 1125; 1032; 996; 909; 861; 824; 699.
RMN$^1$H ($CDCl_3$) : 2,33 (s, 3H, $-OCOCH_3$) ; 3, 88 (s, 3H, OMe) ; 6,01 (d, $J_{2-3}$ = 15, 2 Hz, 1H, $H_2$) ; 6, 84 (dd, $J_{4-5}$ = 15, 5 Hz et $J_{4-3}$ = 10, 1 Hz, 1H, $H_4$) ; 6,92 (d,$J_{5-4}$ = 15,5 Hz, 1H, $H_5$) ; 7,03 (d, $J_{5'-6'}$= 8,1 Hz, 1H, $H_{5'}$) ; 7, 06-7, 09 (m, 2H, $H_{2'}$ et $H_{6'}$) ; 7,52 (dd,$J_{3-2}$ = 15,2 Hz et $J_{3-4}$ = 10, 1 Hz, 1H, $H_3$).
RMN$^{13}$C (DMSO-$d_6$) : 20, 4 ($-OCOCH_3$) ; 55, 9 (OMe); 110,7; 120,1; 122,3; 123,2; 127,0; 135,0; 139,3; 139,9; 144,3; 151,1; 167,6 ($-OCOCH_3$) ; 168,5 (C1).
Masse (ESI) : $[M-H]^+$ 261.

Exemple 23 :

Acétate de 4-{(1E,3E)-5-[(éthoxycarbonyl)oxy]-5-oxo penta-1,3-dièn-1-yl}-2-méthoxyphényle (19)

La réaction est réalisée selon le même protocole que pour l'exemple 2 en utilisant 15g (0,0571 mole) du composé (18). On obtient sans autre purification 13,2 g (Rdt: 69%) du composé (19) sous la forme d'un solide blanc $C_{17}H_{18}O_7$: 334,33.
F: 114°C.
IR (KBr) ν (cm$^{-1}$) : 1800 et 1723 (C=O anhydride); 1763 (C=O ester); 1618; 1508; 1469; 1424; 1371; 1356; 1206; 1164; 1102; 870.
RMN$^1$H ($CDCl_3$) : 1,39 (t, J = 7,4 Hz, 3H, $-OCH_2CH_3$) ; 2, 33 (s, 3H, $-OCOCH_3$) ; 3,88 (s, 3H, OMe); 4,36 (q, J = 7,4 Hz, 2H, $-OCH_2CH_3$) ; 5,98 (d, $J_{2-3}$ = 15,6 Hz, 1H, $H_2$) ; 6,85 (dd, $J_{4-5}$ = 15,1 Hz et $J_{4-3}$ = 10,7 Hz, 1H, $H_4$) ; 6,96 (d, $J_{5-4}$ = 15,6 Hz, 1H, $H_5$); 7,03-7,09 (m, 3H, $H_{2'}$, $H_{5'}$ et $H_{6'}$ ; 7,58 (dd, $J_{3-2}$ = 15,6 Hz et $J_{3-4}$ = 10,7 Hz, 1H, $H_3$).
Masse (ESI) : $[M-H]^+$ 333.

Exemple 24

3-({(2E,4E)-5-[4-(acétyloxy)-3-méthoxyphényl]penta-2,4-diènoyl}amino)-4-méthylthiophène-2-carboxylate méthyle (20)

Un mélange intime de 3 g (0,0089 mole) du composé (19) et 1,54 g (0,009 mole) de 3-amino-4-méthyl thiophène-2-carboxylate de méthyle est chauffé 35 minutes à 130°C. Après refroidissement de la solution, on ajoute 5 ml d'acétonitrile et on laisse agiter 8 heures à température ambiante. Le précipité blanc formé est essoré, lavé avec 25 ml d'éther éthylique et séché. On obtient 2,42 g (Rdt : 65%) du composé (20). $C_{21}H_{21}NO_6S$ : 415,47.
F: 162°C.
IR (KBr) ν (cm$^{-1}$) : 3323 (NH), 1755 (C=O ester acétoxyle), 1696 (C=O ester thiophène), 1667 (C=O amide), 1626, 1616, 1567, 1512, 1503, 1450, 1386, 1299, 1273, 1247, 1215, 1192, 1119, 1028, 1004, 948, 899, 857, 800, 616, 605.
RMN$^1$H (DMSO-$d_6$) : 2,04 (s, 3H, $Me_{thiophène}$) ; 2,25 (s, 3H, -$OCOCH_3$) ; 3,74* (s, 3H, -$CO_2CH_3$) ; 3,82* (s, 3H, -OMe) ; 6,42 (d, $J_{2-3}$ = 14,6 Hz, 1H, $H_2$) ; 7, 01 (d, $J_{4-5}$ = 15,4 Hz, 1H, $H_4$) ; 7,08 (d, $J_{5'-6'}$ = 8,0 Hz, 1H, $H_{5'}$ ) ; 7,13-7,20 (m, 2H, $H_5$ et $H_{6'}$) ; 7,32 (dd, $J_{3-2}$ = 14,6 Hz et $J_{3-4}$ = 10,5 Hz, 1H, $H_3$) ; 7,35 (s, 1H, $H_{2'}$) ; 7,55 (s, 1H, $H_{thiophène}$ ) ; 9,86 (s large, 1H, NH).
RMN$^{13}$C ($CDCl_3$) : 16,0 ($Met_{hiophène}$) ; 20,7 (-$OCO\underline{C}H_3$) ; 52,0 (-$CO_2\underline{C}H_3$) ; 55,9 (-OMe) ; 110,5; 117,2; 120,0; 123,1; 123,7; 126,4; 127,7; 135,2; 135,9; 139,5; 140,3; 142,5; 143,3; 151,2; 163,9* (C1) ; 164,0* (-$\underline{C}O_2CH_3$) ; 169,0 (-$O\underline{C}OCH_3$) .
Masse (ESI) : $[M+H]^+$ 416.

Analyse élémentaire : théorique C 60,71%, H 5,09%, N 3,37%; expérimentale C 60,39%, H 5,02%, N 3,06%.

Exemple 25 :

3-{[(2E,4E)-5-(4-hydroxy-3-méthoxyphényl)penta-2,4-diènoyl]amino}-4-méthylthiophène-2-carboxylate de méthyle (21)

A température ambiante, l'ester (20) [1g, 0,0024 mole] est dissous dans un mélange volume à volume de THF et de méthanol (20/20) en présence de deux équivalents de carbonate de potassium puis le mélange réactionnel est chauffé à 40°C pendant 2 heures. Les solvants sont évaporés, et le résidu est repris par une solution d'acide cholhydrique 0,5 N. La phase aqueuse est extraite par 100 ml de chloroforme, séchée puis évaporée sous pression réduite. On obtient sans autre purification 0,70 g (Rdt = 72%) du composé (21) sous la forme d'une poudre jaune. $C_{19}H_{19}NO_5S$ : 373,43.
F: 162°C.
IR(KBr)ν(cm$^{-1}$) : 3415 (O-H) : 1702 (C=O ester); 1639 (C=O amide); 1595; 1513; 1451; 1388; 1349; 1257; 1206; 1130, 1067; 1031; 1001; 862; 797.
RMN$^1$H ($CDCl_3$) : 2,26 (s, 3H, $Me_{thiophène}$) ; 3,87* (s, 3H, -$CO_2CH_3$) ; 3,95* (s, 3H, -OMe); 5,78 (s, 1H, OH); 6,18 (d, $J_{2-3}$ = 14,9 Hz, 1H, $H_2$) ; 6,78 (dd, $J_{4-5}$ = 15,4 Hz et $J_{4-3}$ = 10,2 Hz, 1H, $H_4$) ; 6,86 (d, $J_{5-4}$ = 15,4 Hz, 1H, $H_5$) ; 6,91 (d, $J_{5'-6'}$ = 8,3 Hz, 1H, $H_{5'}$ ) ; 6, 98 (d, $J_{2'-6'}$ = 2,0 Hz, 1H, $H_{2'}$) ; 7,02 (dd, $J_{6'-5'}$ = 8,3 Hz et $J_{6'-2'}$ = 2,0 Hz, 1H, $H_{6'}$) ; 7,16 (s, 1H, $H_{thiophène}$) ; 7,48 (dd, $J_{3-2}$ = 14,9 Hz et $J_{3-4}$ = 10,2 Hz, 1H, $H_3$) ; 9,02 (s large, 1H, NH) .
RMN$^{13}$C ($CDCl_3$) : 16,2 ($Me_{thiophène}$) ; 52,1 (-$CO_2\underline{C}H_3$) ; 56,1 (-OMe); 108, 9; 114, 9; 121, 7; 122, 4; 124, 2; 127, 9; 129, 0; 136,1; 140,6; 143,3; 143,7; 146,9; 147,0; 164,1* (C1) ; 164,5* (-$\underline{C}O_2CH_3$). (Un signal est manquant) .
Masse (ESI) : $[M+H]^+$ 374.

Analyse élémentaire : théorique C 61,11%, H 5,13%, N 3,75%; expérimentale C 61,49%, H 5,26%, N 3,49%.

Exemple 26 :

Acide 3-{[(2E,4E)-5-(4-hydroxy-3-méthoxyphényl)penta-2,4-diènoyl]amino}-4-méthylthiophène-2-carboxylique (22)

Le dérivé ester (20) [1g, 0,0024 mole] est mis en solution dans 20 ml d'un mélange THF/$H_2O$ (1/1). 0,13 g (0,0053 mole) de LiOH est ajouté et le milieu réactionnel est chauffé à 50°C pendant 1H30.

La solution est refroidie à 0°C puis acidifiée par une solution aqueuse d'acide chlorhydrique 1N jusqu'à pH = 2.

Le précipité formé est filtré sur fritté puis lavé à l'acétonitrile. On obtient sans autre purification 0,67g (Rdt= 78%) du composé (22) sous la forme d'une poudre jaune pâle.$C_{18}H_{17}NO_5S$ : 359,40.
F: 248°C.
IR(KBr)ν(cm$^{-1}$) : 3508 et 3349 (N-H et O-H); 1683 (C=O acide), 1651 (C=O amide), 1598, 1563, 1510, 1496, 1450, 1432, 1374, 1277, 1251, 1201, 1152, 1145, 1024, 994, 853, 808, 723, 665, 560.
RMN$^1$H (DMSO-$d_6$) : 2,03 (s, 3H, $Me_{thiophène}$) ; 3,81 (s, 3H, -OMe) ; 6,31 (d, $J_{2-3}$ = 14,7 Hz, 1H, $H_2$) ; 6, 75-6, 99 (m, 4H, $H_4$, $H_5$, $H_{5'}$ et $H_{6'}$) ; 7,17 (s, 1H, $H_{2'}$) ; 7,28 (dd, $J_{3-2}$ = 14,9 Hz et $J_{3-4}$ = 10,2 Hz, 1H, $H_3$) ; 7,45 (s, 1H, $Ht_{hiophène}$) ; 9,39 (s large, 1H, NH); 9,74 (s large 1H, OH).
RM$^{13}$C (DMSO-$d_6$) : 14,5 ($Me_{thiophène}$) ; 55,7 (-OMe); 110,4 ; 115,6; 121,3; 122,6; 123,8; 126,3 (2C); 127,9; 136,6; 139,8; 140,7; 141,6; 147,8; 147,9; 162,9 (C1) ; 163,9 ($CO_2H$) .

Masse (ESI): $[M+H]^+$ 360.

Exemple 27:

3-{[(2E,4E)-5-(4-hydroxy-3-méthoxyphényl)penta-2,4-diènoyl]amino}-4-méthylthiophène-2-carboxylate d'éthyle (23)

On porte au reflux 4 heures dans 60 ml d'éthanol absolu en présence de 0,05 ml d'acide sulfurique, 0,3g (0,00083 mole) du dérivé acide (22). Après refroidissement, la solution est concentrée sous pression réduite, le résidu est repris par 40 ml d'eau froide puis extrait par 70 ml d'acétate d'éthyle. La phase organique est séchée, concentrée sous vide ; le résidu est cristallisé dans un mélange éther/acétonitrile (3/1). Poudre jaunâtre, poids: 0,16g (Rdt= 50%) F: 158°C.
IR (KBr) $\nu$ ($cm^{-1}$) 3415 (OH, NH), 1700 (CO), 1640 (CO).
RMN$^1$H ($CDCl_3$) : 1,08(t, 3H, $CO_2CH_2\underline{CH_3}$) ; 2,26 (s, 3H, $CH_{3thiophène}$) ; 3,40 (q, 2H, $CO_2\underline{CH_2}CH_3$) ; 3,90 (s, 3H, $OCH_3$) ; 5,70 (s, 1H, OH); 6,18 (d, $J_{2\text{-}3}$ = 14,8 Hz, 1H, $H_2$) ; 6,76 (dd, $J_{4\text{-}5}$ = 15Hz et $J_{4\text{-}3}$ = 10,4 Hz, 1H, $H_4$) ; 6,80 (d, $J_{5\text{-}4}$ = 15 Hz, 1H, $H_5$) ; 6, 90 (d, $J_{5'\text{-}6'}$ = 8,3 Hz, 1H, $H_5$) ; 6, 96 (d, $J_{2'\text{-}6'}$ = 2 Hz, 1H, $H_{2'}$) ; 7,02 (dd, $J_{6'\text{-}5'}$ = 8,3 Hz et $J_{6'\text{-}2'}$ = 2 Hz, 1H, $H_{6'}$) ; 7,20 (s, 1H, $H_{thiophène}$) ; 7,48 (dd, $J_{3\text{-}2}$ = 14,9 Hz et $J_{3\text{-}4}$ = 10,2 Hz, 1H, $H_3$) ; 9,10 (s, 1H, NH) .
Masse (ESI) : $[M+H]^+$ : 388.
$C_{20}H_{21}NO_5S$ : 387,38.

Exemple 28:

N,N'-diéthyl-3-{[(2E,4E)-5-(4-hydroxy-3-méthoxyphényl)penta-2,4-diènoyl]amino}-4-méthylthiophène-2-carboxamide (24)

On agite à 0°C, 0,5g (0,00139 mole) d'acide (22) dans 60ml d'acétonitrile puis on ajoute goutte à goutte 0,194 ml de triéthylamine et on laisse 30 minutes à cette température. On additionne ensuite 0,133ml de chloroformiate d'éthyle et on poursuit l'agitation 1H30. Le chlorhydrate de triéthylammonium formé est filtré sur fritté et la solution organique est additionnée d'un excès de diéthylamine (4 équivalents) puis chauffée 1H30 à 80°C. Après refroidissement, la solution est concentrée sous pression réduite, le résidu formé est additionné de 50 ml d'eau et extrait par 2 fois 50 ml d'acétate d'éthyle.

La phase organique est séchée sur sulfate de magnésium, puis évaporée. Le résidu est purifié dans l'acétonitrile. Poudre jaune, F: 190°C, poids: 0,2g, Rdt : 34%.
IR(KBr)$\nu$($cm^{-1}$) : 3400, 3300 (OH, NH), 1670, 1660 (C=O).
RMN$^1$H (DMSO-$d_6$) : 1,10 (t, 6H, $(CH_2CH_3)_2$ ; 2,08 (s, 3H, $CH3_{thiophène}$) ; 3,38 (q, 4H, $CH_2CH_3)_2$ ; 3,81 (s, 3H, $OCH_3$) ; 6,40 (d, $J_{2\text{-}3}$ = 14, 5 Hz, 1H, $H_2$) ; 6, 70, 7, 00 (m, 4H, $H_4$, $H_5$, $H_5$' et $H_{6'}$) ; 7,10 (s, 1H, $H_{2'}$) ; 7,24 (dd, $J_{3\text{-}2}$ = 14,7 Hz et $J_{3\text{-}4}$ = 10 Hz, 1H, $H_3$) ; 7,42 (s, 1H $H_{thiophène}$) ; 9,34 (s, 1H, OH) ; 9,74 (s, 1H, NH).
Masse (ESI) : $[M+H]^+$ 415.
$C_{22}H_{26}N_2O_4S$ : 414,44.

Les composés selon l'invention présentent des propriétés apaisantes, anti-radicaux libres, antioxydantes et protectrices contre les UV et trouvent, de ce fait, leur utilisation dans la fabrication de compositions cosmétiques.

La présente invention s'étend donc à des compositions cosmétiques comprenant au moins l'un des dérivés de formule (I), isomères et sels ci-dessus, en association avec un support cosmétiquement acceptable.

Les compositions cosmétiques susvisées sont utilisables pour la prévention et le traitement du vieillissement, notamment de la peau, en raison de leurs activités antioxydantes, anti-radicaux libres et protectrices contre les UV ; elles sont en outre utilisables pour apaiser la peau et/ou le cuir chevelu, à savoir notamment prévenir l'apparition ou réduire l'intensité d'inconforts cutanés légers, tels que pellicules, rougeurs, démangeaisons, sensibilités, gonflement, brûlures légères (feu du rasoir) et irritations.

On décrit ci-après la mesure de l'activité antioxydante de dérivés de formule (I), isomères et sels de la présente invention.

Activité antioxydante

Les espèces réactives de l'oxygène (ERO) et les radicaux libres sont connus pour être impliqués dans la pathogénèse du vieillissement et de certaines maladies humaines comme le cancer et l'athérosclérose. Ces substances, telles que l'anion superoxyde ($O_2^{\cdot-}$), le peroxyde d'hydrogène ($H_2O_2$) et le radical hydroxyle ($OH\cdot$), sont naturellement produites en permanence dans le corps humain. Leur action est limitée par des systèmes de défense antioxydants (enzymatiques et non enzymatiques).

Le stress oxydatif, qui résulte d'un déséquilibre de la balance entre pro-oxydants et anti-oxydants, est responsable de lésions tissulaires et du processus de vieillissement.

Des dérivés selon l'invention ont été testés par des méthodes in vitro. Deux types de tests ont été mis en oeuvre :

- un test visant à mesurer la capacité à piéger des radicaux libres (test au DPPH),
- un test d'évaluation du pouvoir antioxydant par la méthode de peroxydation lipidique induite.

1 - Evaluation par piégeage du radical DPPH (2,2-diphényl-1-picrylhydrazyle)

Cette étude mesure l'activité antiradicalaire d'un composé par capture du radical DPPH. Ce test est largement utilisé pour étudier la capacité d'un composé à donner un hydrogène.

Le principe de cette méthode consiste à mettre en solution le DPPH (0,5mM-DO~1,2) en présence du dérivé à tester à différentes concentrations (80, 40 et 25μM) et à 37°C pendant 30 minutes. L'activité se traduit par une décoloration du DPPH (violet). L'absorbance (densité optique DO) a été mesurée à 517 nm au bout de trente minutes sur un spectrophotomètre UV-visible thermostaté à 37°C. Le résultat est exprimé en pourcentage d'inhibition déterminé par la formule :

$$\%\ d'inhibition = \frac{A_{contrôle} - A_{échantillon}}{A_{contrôle}}$$

Cette activité a été comparée à celle des produits de référence vitamine E, acide férulique et vinylogue de l'acide férulique [dérivé (17)].

Les résultats sont présentés dans le Tableau 3 ci-après.

seuls les composés 21 et 23 sont compris dans l'invention.

Tableau 3

| Composé | % inhibition (C=80μM) | % inhibition (C=50μM) | % inhibition (C=25μM) |
|---|---|---|---|
| 3 | 72 | 48 | 45 |
| 4 | 50 | 31 | 41 |
| 21 | 93 | 74 | 68 |
| 22 | 73 | 54 | 50 |
| Acide férulique | 78 | 61 | 50 |
| 17 | 81 | 65 | 62 |
| Vit.E | 92 | 86 | 41 |

D'après ces résultats, on constate tout d'abord que les dérivés en série avénalumique (21, 22) sont plus actifs que ceux en série férulique (3, 4). Le composé 21 possède des pourcentages d'inhibition supérieurs à ceux du dérivé (17) et ce, aux trois concentrations étudiées.

Par ailleurs, la présence de la fonction ester du composé (21) joue un rôle essentiel dans l'activité antiradicalaire par rapport à l'acide correspondant (22).

On remarque également que tous les dérivés testés, excepté le dérivé (4), ont une activité de piégeage de radicaux meilleure que celle de la vitamine E, à la concentration de 25 μM.

2 - Evaluation de l'auto-oxydation par la méthode du thiocyanate ferrique (FTC)

Cette méthode met en évidence la capacité de capter le radical peroxyle par le dérivé à tester. La méthode est basée sur une peroxydation lipidique de l'acide linoléique, émulsionné par le Tween 20®.

Cette peroxydation est réalisée par action d'un initiateur de radicaux libres, le dihydrochlorhydrate de 2,2'-azobis(2-amidino)propane (AAPH). Le radical peroxyle formé réagit avec le dérivé à tester et conduit à la formation d'un hydroperoxyde et d'un radical alkoxyle ArO˙. Ce dernier réagit avec le radical peroxyle et permet la formation de produits non radicalaires.

Par ailleurs, les radicaux peroxyle continuent d'oxyder le substrat (l'acide linoléique) pour former également un hydroperoxyde. Cet hydroperoxyde oxyde le fer ferreux présent in situ pour conduire à la formation de fer ferrique

$Fe^{3+}$ qui se complexe à l'anion thiocyanate.

Le complexe rouge de thiocyanate ferrique formé absorbe à 500nm.

Le degré d'oxydation est quantifié par l'absorbance mesurée. L'activité antioxydante du dérivé à tester est donc la capacité à retarder la peroxydation lipidique. Plus la durée pour atteindre le maximum d'absorbance est longue, plus l'antioxydant est puissant.

Cette étude est réalisée sur une période de cinq heures avec un intervalle de temps d'une heure. Le résultat est exprimé par l'absorbance en fonction du temps. La concentration des molécules dans le milieu est de 25 micromoles.

Cette étude, effectuée sur les dérivés 3, 4, 17, 21 et 22 et sur l'acide férulique, montre que les dérivés 3, 4, 21 et 22 ont une activité antioxydante, laquelle n'est cependant pas supérieure à celle de l'acide férulique.

Les dérivés, isomères et sels selon l'invention sont également réputés posséder une activité apaisante.

Cette activité est déduite des relations structure/activité concernant les anesthésiques locaux dont les structures chimiques dérivent d'amides benzoïques, anthraniliques et de leurs homologues thiophéniques.

En particulier, d'une part sur la base de la structure de la lidocaïne et de la prilocaïne qui sont les deux principes actifs contenus dans la crème anesthésique dermique bien connue EMLA, et d'autre part sur la base de la structure chimique de l'anesthésique local la carticaïne qui est un principe actif de choix des préparations pharmaceutiques utiles en odontologie, ORL et ophtalmologie et compte tenu de plus des requis structuraux qui définissent le pharmacophore des anesthésiques locaux, il peut être avancé que les composés de l'invention présenteront une activité légère d'anesthésique de surface qui pourra conférer aux préparations cosmétiques les contenant une activité apaisante.

Compte tenu des concentrations auxquelles sont utilisés les anesthésiques locaux cités plus haut, l'activité apaisante des compositions cosmétiques contenant les composés de l'invention devrait se manifester pour des concentrations de l'ordre de 0,5 à 5% en poids desdites compositions.

Les concentrations ci-dessus seront de même suffisantes pour obtenir les autres effets recherchés (anti-radicaux libres, antioxydation, protection contre les UV) des composés selon l'invention.

Ces compositions cosmétiques sont administrées notamment par voie topique sur la peau et le cuir chevelu, sous forme de crèmes, pommades, gels, lotions, shampoings, mousse à raser.

Le support mis en oeuvre dans ces compositions est fonction de la forme adoptée pour ces dernières ; il peut être aqueux ou non aqueux et comprendre des adjuvants choisis parmi ceux classiquement utilisés dans les produits cosmétiques, tels que alcool éthylique, propylèneglycol, tensioactifs, agents épaississants, agents gélifiants et conservateurs.

On donnera ci-après quelques exemples non limitatifs de compositions selon l'invention.

Il s'agit plus précisément de compositions contenant 1, 2 et 5% en poids du composé n°4 selon l'invention.

Composition N°1 : Crème hydrophile H/E

| | % | Constituants (nomenclature INCI) | Catégorie |
|---|---|---|---|
| A | 2,00 | ceteareth-6, stearyl alcohol | tensio-actif non ionique |
| | 2,00 | ceteareth-25 | tensio-actif non ionique |
| | 4,00 | cetearyl alcohol | alcool gras |
| | 10,00 | cetearyl octanoate | tensio-actif non ionique |
| | 3, 00 | glyceryl stearate | émollient, émulsionnant |
| | 5, 00 | petrolatum | conditionneur, épaississant, protecteur |
| | | | |
| B | 0,20 | EDTA | chélatant |
| | 5, 00 | propylene glycol | humectant, solvant |
| | q.s. | conservateur | |
| | q.s.p. 100,00 | aqua | |
| | | | |
| C | 1 (ou 2 ou 5) | Substance active : Composé n°4 | |

(suite)

| | % | Constituants (nomenclature INCI) | Catégorie |
|---|---|---|---|
| Chauffer les phases A et B séparément à 80°C. Ajouter et mélanger la phase aqueuse B à la phase grasse A et homogénéiser parfaitement. Refroidir le mélange à 40°C et ajouter la phase C et homogénéiser. | | | |

Composition N°2 : Crème hydrophile H/E

| | % | Constituants (nomenclature INCI) | Catégorie |
|---|---|---|---|
| A | 6, 00 | PEG-7 hydrogenated castor oil | corps gras, conditionneur, épaississant |
| | 10,00 | minéral oil | conditionneur, émollient |
| | 3, 00 | petrolatum | conditionneur, épaississant, protecteur |
| | 5, 00 | caprylic/capric triglycerides | corps gras, émollient, conditionneur |
| | 2,00 | PEG-45/Dodecyl glycol copolymer | tensio-actif non ionique |
| | 5, 00 | jojoba oil | huile végétale, régénérant, réparateur conditionneur |
| | 1,00 | quaternium-18 bentonite | absorbant, épaississant |
| | | | |
| B | 0,10 | EDTA | chélatant |
| | 3, 00 | propylene glycol | humectant, solvant |
| | q.s. | preservative | |
| | q.s.p. 100,00 | aqua | |
| | | | |
| C | 1 (ou 2 ou 5) | Substance active : composé n°4 | |
| Chauffer les phases A et B séparément à 80°C. Ajouter et mélanger la phase aqueuse B à la phase grasse A et homogénéiser parfaitement. Refroidir le mélange à 40°C et ajouter la phase C et homogénéiser. | | | |

Composition N° 3: Pommade

| | % | Constituants (nomenclature INCI) | Catégorie |
|---|---|---|---|
| A | 7,00 | petrolatum | cire minérale, conditionneur, épaississant, protecteur |
| | 13,00 | microcristalline wax | cire minérale, émollient, protecteur |
| | q.s.p. 100,00 | paraffinum liquidum | émolliente, protectrice |
| | | | |
| | 1 (ou 2 ou 5) | Substance active : composé n°4 | |
| Au bain marie, faire fondre les cires minérales dans la paraffine liquide ; quand les cires sont fondues, retirer du bain marie et bien remuer le mélange jusqu'à ce qu'il commence à prendre, ensuite ajouter le composé n°4 et bien mélanger. | | | |

Composition N°4 : Pommade

| | % | Constituants (nomenclature INCI) | Catégorie |
|---|---|---|---|
| A | 25, 00 | beeswax | stabilisateur d'émulsion, conditionneur, épaississant |
| | q.s.p. 100,00 | mineral oil | conditionneur, émolliente |
| | | | |
| | 1 (ou 2 ou 5) | Substance active : composé n°4 | |
| Au bain marie, faire fondre la cire d'abeille dans l'huile minérale ; quand la cire est fondue, retirer du bain marie et bien remuer le mélange jusqu'à ce qu'il commence à prendre, ensuite ajouter le composé n°4 et bien mélanger. | | | |

**Revendications**

1. Dérivés amides de formule générale :

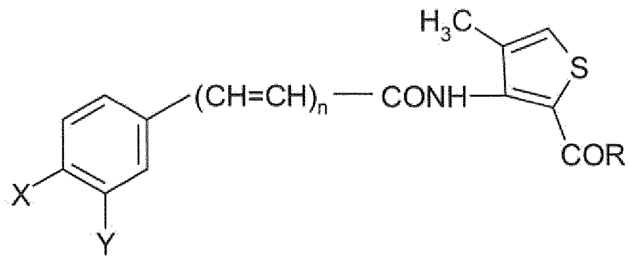

(I)

dans laquelle :

- n = 2,
- le couple (X, Y) est le couple (OH, $OCH_3$)
- R représente un groupe OH, un groupe OR' où R' est un groupe alkyle linéaire ou ramifié, en $C_1$-$C_{12}$, ou un groupe $NR_1R_2$ où $R_1$ et $R_2$ sont chacun indépendamment un atome d'hydrogène, un groupe alkyle linéaire ou ramifié, en $C_1$-$C_6$, ou un groupe cycloalkyle en $C_3$-$C_8$ ramifié ou non,

ainsi que leurs isomères ayant la même formule structurelle que celle définie par la formule (I) ci-dessus et leur sels.

2. Dérivés, isomères et sels selon la revendication 1, de formule (I) dans laquelle R a les significations suivantes :

- OH, $OCH_3$, $OC_2H_5$, O-n$C_3H_7$, O-iso$C_3H_7$, O-n$C_4H_9$, O-iso $C_4H_9$, O-tert.$C_4H_9$ ou $O(CH_2)_{11}$-$CH_3$.

3. Dérivés, isomères et sels selon la revendication 2, de formule (I), dans laquelle :

- R=$NR_1R_2$ où le couple ($R_1$,$R_2$)=(H,H), (H,$CH_3$), (H, n$C_3H_7$), (H,iso$C_3H_7$), (H,n$C_4H_9$), (H,iso$C_4H_9$) ou (H,tert$C_4H_9$).

4. Dérivé selon la revendication 1, constitué par l'acide 3-{[(2E)-3-(4-hydroxy-3-méthoxyphényl)prop-2-ènoyl]amino}-4-méthylthiophène-2-carboxylique.

5. Procédé de préparation des dérivés, isomères et sels selon la revendication 1, **caractérisé en ce qu'**il comprend :

(i) la réaction des composés de formule:

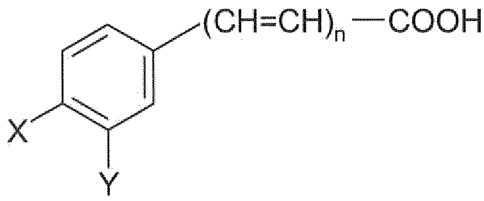

(II)

dans laquelle X, Y et n ont les mêmes significations que dans la revendication 1, avec le 4-méthyl-3-aminothiophène-2-carboxylate de méthyle de formule :

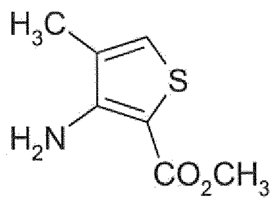

(III)

pour obtenir les dérivés de formule :

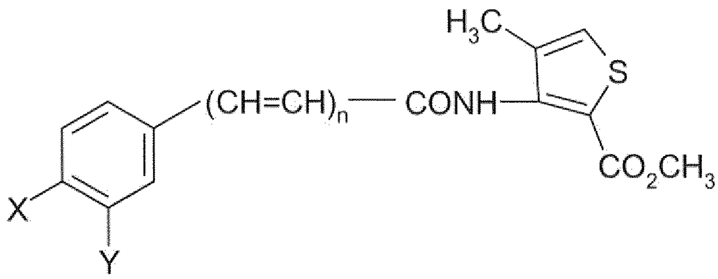

(ii) la conversion par hydrolyse du groupe $CO_2CH_3$ présent sur le noyau tiophène des dérivés obtenus à l'étape (i), en un groupe carboxyle, pour obtenir des dérivés de formule (I) dans laquelle R=OH,
(iii) l'estérification éventuelle du groupe carboxyle des dérivés (I) obtenus à l'étape (ii), par un alcool R'OH où R' a la même signification que dans la revendication 1, pour obtenir les dérivés de formule (I) dans laquelle R=OR',
(iv) l'amidation éventuelle des dérivés de formule (I) obtenus à l'étape (ii) par un composé $HNR_1R_2$ où $R_1$ et $R_2$ ont la même signification que dans la revendication 1, pour obtenir les dérivés de formule (I) dans laquelle $R=NR_1R_2$, et
(v) la salification éventuelle des dérivés de formule (I) obtenus à l'étape (ii) par une base minérale ou organique appropriée.

**6.** Procédé selon la revendication 5, dans lequel X dans la formule (I) représente le groupe OH, ce dernier groupe étant protégé, avant la réaction (i) ci-dessus, par un groupe protecteur, tel qu'un groupe $CH_3CO$, la réaction (i) étant suivie de la déprotection dudit groupe OH.

**7.** Procédé selon la revendication 5 ou 6, dans lequel, lors de la réaction (i), le groupe carboxyle du composé de formule (II) est mis en oeuvre sous une forme activée, telle que la forme d'un anhydride mixte.

**8.** Utilisation des dérivés, isomères et sels selon l'une quelconque des revendications 1 à 4 pour la préparation d'une composition cosmétique.

**9.** Utilisation cosmétique selon la revendication 8, **caractérisée en ce que** la composition cosmétique est destinée à prévenir et traiter le vieillissement cutané

**10.** Dérivés amides de formule (I) selon une quelconque des revendications 1 à 4 destinés à apaiser la peau et/ou le cuir chevelu.

**11.** Composition cosmétique comprenant au moins un dérivé, isomère ou sel selon l'une quelconque des revendications 1 à 4 et un véhicule acceptable sur le plan cosmétique.

**12.** Composition cosmétique selon la revendication 11, destinée à prévenir et traiter le vieillissement cutané

**13.** Composition comprenant les dérivés amides de formule (I) selon l'une quelconque des revendications 1 à 4 destinée à apaiser la peau et/ou le cuir chevelu contre des inconforts cutanés légers, tels que pellicules, rougeurs, démangeaisons, sensibilités, gonflements, brûlures légers et irritations.

**14.** Intermédiaires de synthèse de formule suivante :

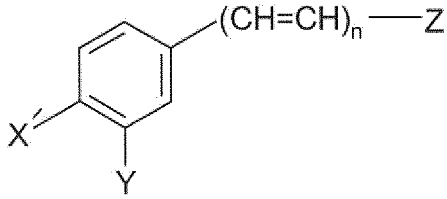

dans laquelle X', Y, n et Z ont les significations suivantes :

| X' | Y | n | Z |
|---|---|---|---|
| $CH_3COO$ | $CH_3O$ | 2 | $COO\text{-}COOC_2H_5$ |
| $CH_3COO$ | $CH_3O$ | 2 | 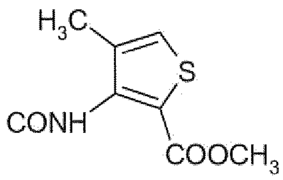 |

**Patentansprüche**

**1.** Amidderivate der allgemeinen Formel:

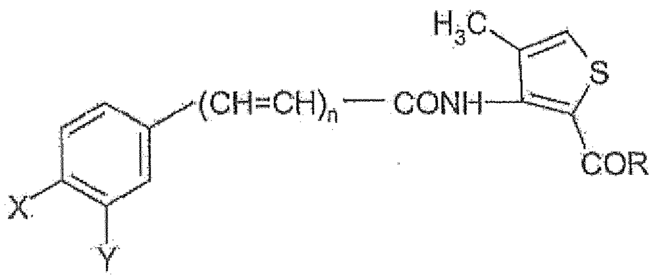

(I)

in der:

- n = 2,
- das Paar (X, Y) das Paar (OH, $OCH_3$) ist,
- R für eine OH-Gruppe, eine OR'-Gruppe, wobei R' eine lineare oder verzweigte $C_1$-$C_{12}$-Alkylgruppe ist, oder eine $NR_1R_2$-Gruppe, wobei $R_1$ und $R_2$ jeweils unabhängig ein Wasserstoffatom, eine lineare oder verzweigte

$C_1$-$C_6$-Alkylgruppe oder eine verzweigte oder unverzweigte $C_3$-$C_8$-Cycloalkylgruppe sind, steht,

sowie Isomere davon mit der gleichen Strukturformel wie durch die obige Formel (I) definiert und Salze davon.

**2.** Derivate, Isomere und Salze nach Anspruch 1 der Formel (I), in der R die folgenden Bedeutungen hat:

- OH, $OCH_3$, $OC_2H_5$, O-n-$C_3H_7$, O-iso-$C_3H_7$, O-n-$C_4H_9$, O-iso-$C_4H_9$, O-tert.-$C_4H_9$ oder $O(CH_2)_{11}$-$CH_3$.

**3.** Derivate, Isomere und Salze nach Anspruch 2 der Formel (I), in der:

- R = $NR_1R_2$, wobei das Paar ($R_1$, $R_2$) = (H, H), (H, $CH_3$), (H, n-$C_3H_7$), (H, iso-$C_3H_7$), (H, n-$C_4H_9$), (H, iso-$C_4H_9$) oder (H, tert-$C_4H_9$).

**4.** Derivat nach Anspruch 1, bestehend aus 3-{[(2E)-3-(4-Hydroxy-3-methoxyphenyl)prop-2-enoyl]amino}-4-methylthiophen-2-carbonsäure.

**5.** Verfahren zur Herstellung der Derivate, Isomere und Salze nach Anspruch 1, **dadurch gekennzeichnet, dass** es Folgendes umfasst:

(i) Umsetzung der Verbindungen der Formel:

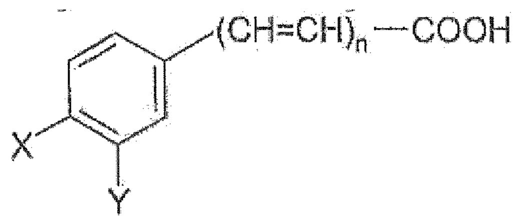

(II)

in der X, Y und n die gleichen Bedeutungen wie in Anspruch 1 haben, mit 4-Methyl-3-aminothiophen-2-carbonsäuremethylester der Formel:

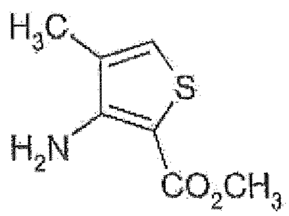

(III)

zum Erhalt der Derivate der Formel:

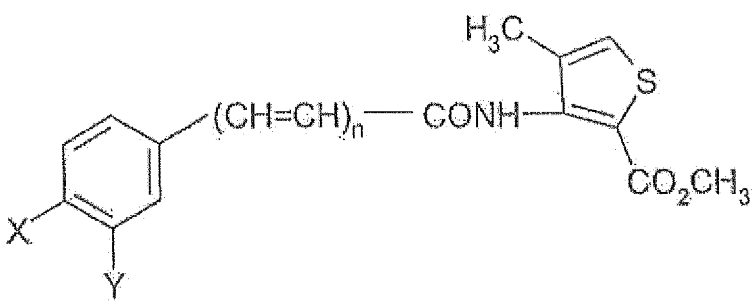

(ii) Umwandlung der $CO_2CH_3$-Gruppe am Thiophenkern der in Schritt (i) erhaltenen Derivate in eine Carboxylgruppe durch Hydrolyse zum Erhalt der Derivate der Formel (I), in der R = OH,

(iii) gegebenenfalls Veresterung der Carboxylgruppe der in Schritt (ii) erhaltenen Derivate (I) mit einem Alkohol R'OH, wobei R' die gleiche Bedeutung wie in Anspruch 1 hat, zum Erhalt der Derivate der Formel (I), in der R = OR',

(iv) gegebenenfalls Amidierung der in Schritt (ii) erhaltenen Derivate der Formel (I) mit einer Verbindung $HNR_1R_2$, wobei $R_1$ und $R_2$ die gleiche Bedeutung wie in Anspruch 1 haben, zum Erhalt der Derivate der Formel (1), in der R = $NR_1R_2$, und

(v) gegebenenfalls Versalzung der in Schritt (ii) erhaltenen Derivate der Formel (I) mit einer geeigneten anorganischen oder organischen Base.

6. Verfahren nach Anspruch 5, bei dem X in der Formel (I) für die OH-Gruppe steht, wobei letztere Gruppe vor der obigen Umsetzung (i) mit einer Schutzgruppe, wie einer $CH_3CO$-Gruppe, geschützt wird und die OH-Gruppe nach der Umsetzung (i) entschützt wird.

7. Verfahren nach Anspruch 5 oder 6, bei dem bei der Umsetzung (i) die Carboxylgruppe der Verbindung der Formel (II) in eine aktivierte Form, wie die Form eines gemischten Anhydrids, gebracht wird.

8. Verwendung der Derivate, Isomere und Salze nach einem der Ansprüche 1 bis 4 zur Herstellung einer kosmetischen Zusammensetzung.

9. Kosmetische Verwendung nach Anspruch 8, **dadurch gekennzeichnet, dass** die kosmetische Zusammensetzung zur Prävention und Behandlung von Hautalterung bestimmt ist.

10. Amidderivate der Formel (I) nach einem der Ansprüche 1 bis 4 zur Beruhigung der Haut und/oder der Kopfhaut.

11. Kosmetische Zusammensetzung, umfassend mindestens ein Derivat, Isomer oder Salz nach einem der Ansprüche 1 bis 4 und ein kosmetisch unbedenkliches Vehikel.

12. Kosmetische Zusammensetzung nach Anspruch 11 zur Prävention und Behandlung von Hautalterung.

13. Zusammensetzung, umfassend die Amidderivate der Formel (I) nach einem der Ansprüche 1 bis 4 zur Beruhigung der Haut und/oder der Kopfhaut gegenüber leichten Hautbeschwerden, wie Schuppen, Rötungen, Juckreizen, Empfindlichkeiten, Schwellungen, leichten Verbrennungen und Reizungen.

14. Synthesezwischenprodukte der folgenden Formel:

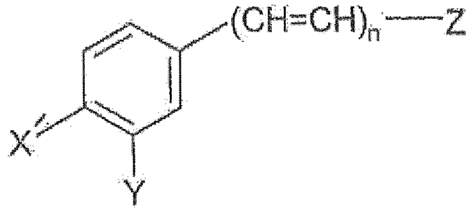

in der X', Y, n und Z die folgenden Bedeutungen haben:

| X' | Y | n | Z |
|---|---|---|---|
| $CH_3COO$ | $CH_3O$ | 2 | $COO\text{-}COOC_2H_5$ |
| $CH_3COO$ | $CH_3O$ | 2 | 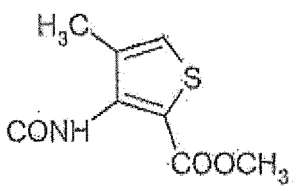 |

**Claims**

1. Amide derivatives of the general formula:

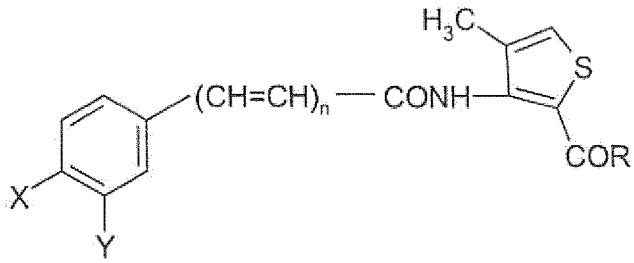

(I)

in which;

- n = 2,
- the pair (X, Y) is the pair (OH, $OCH_3$)
- R represents an OH group, an OR' group where R' is a linear or branched $C_1$-$C_{12}$ alkyl group, or an $NR_1R_2$ group where $R_1$ and $R_2$ are each independently a hydrogen atom, a linear or branched $C_1$-$C_6$ alkyl group, or a branched or unbranched, $C_3$-$C_8$ cycloalkyl group,

together with the isomers thereof having the same structural formula as that defined by the formula (I) above and the salts thereof.

2. Derivatives, isomers and salts according to claim 1, of formula (I) in which R has the following meanings:

- OH, $OCH_3$, $OC_2H_5$, O-n$C_3H_7$, O-iso$C_3H_7$, O-n$C_4H_9$, O-iso$C_4H_9$, O-tert.$C_4H_9$ or $O(CH_2)_{11}$-$CH_3$.

3. Derivatives, isomers and salts according to claim 2, of formula (I), in which:

- R=$NR_1R_2$ where the pair ($R_1$,$R_2$) = (H,H), (H,$CH_3$), (H,n$C_3H_7$), (H,iso$C_3H_7$), (H,n$C_4H_9$), (H,iso$C_4H_9$) or (H,tert.$C_4H_9$).

4. A derivative according to claim 1, constituted by 3-{[{2E)-3-(4-hydroxy-3-methoxyphenyl)prop-2-enoyl]amino}-4-methylthiophene-2-carboxylic acid.

5. A method for preparing the derivatives, isomers and salts according to claim 1, **characterised in that** it involves:

(i) reacting the compounds of formula:

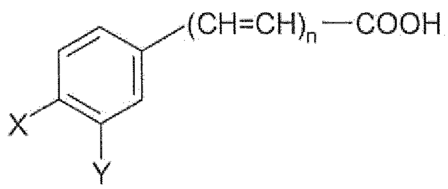

(II)

in which X, Y and n have the same meanings as in claim 1, with methyl 4-methyl-3-aminothiophene-2-carboxylate of formula:

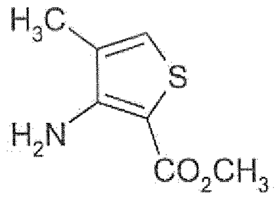

(III)

to obtain derivatives of formula:

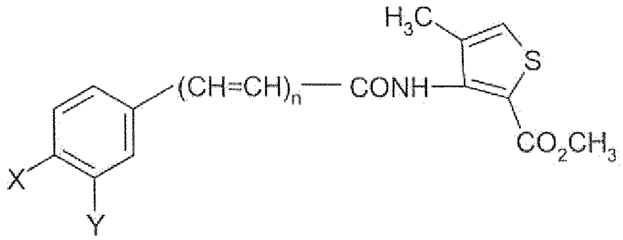

(ii) converting the $CO_2CH_3$ group present on the thiophene nucleus of the derivatives obtained in step (i) by hydrolysis into a carboxyl group to obtain derivatives of formula (I) in which R=OH,
(iii) optionally esterifying the carboxyl group of the derivatives (I) obtained in step (ii), with an alcohol R'OH where R' has the same meaning as in claim 1, to obtain derivatives of formula (I) in which R=OR',
(iv) optionally amidating the derivatives of formula (I) obtained in step (ii) with a compound $HNR_1R_2$ where $R_1$ and $R_2$ have the same meaning as in claim 1, to obtain derivatives of formula (I) in which R-$NR_1R_2$, and
(v) optionally salifying the derivatives of formula (I) obtained in step (ii) with an appropriate inorganic or organic base.

**6.** A method according to claim 5, in which X in formula (I) represents the OH group, the latter group being protected, prior to reaction (i) above, by a protective group, such as a $CH_3CO$ group, reaction (i) being followed by deprotection of said OH group.

**7.** A method according to claim 5 or 6, in which, during reaction (i), the carboxyl group of the compound of formula (II) is used in an activated form, such as a mixed anhydride form.

**8.** Use of the derivatives, isomers and salts according to any one of claims 1 to 4 for preparing a cosmetic composition.

**9.** Cosmetic use according to claim 8, **characterised in that** the cosmetic composition is intended for preventing and treating dermal ageing.

**10.** Amide derivatives of formula (I) according to any one of claims 1 to 4 intended for soothing the skin and/or scalp.

**11.** A cosmetic composition comprising at least one derivative, isomer or salt according to any one of claims 1 to 4 and a cosmetically acceptable vehicle.

**12.** A cosmetic composition according to claim 11 intended for preventing and treating dermal ageing.

**13.** A composition comprising the amide derivatives of formula (I) according to any one of claims 1 to 4 intended for soothing the skin and/or scalp in response to minor dermal disorders, such as dandruff, redness, itching, sensitivity, swelling, minor burns and irritation.

**14.** Synthesis intermediates of the following formula:

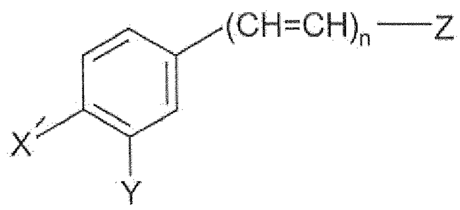

in which X', Y, n and Z have the following meanings:

| X' | Y | n | Z |
|---|---|---|---|
| $CH_3COO$ | $CH_3O$ | 2 | $COO\text{-}COOC_2H_5$ |
| $CH_3COO$ | $CH_3O$ | 2 | 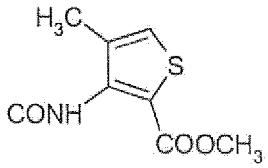 |

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0227431 A


### Littérature non-brevet citée dans la description


- **EBENEZER, W.J.** *Synth. Commun.,* 1991, vol. 21 (3), 351-8
- *J. Mol. Catal.B : Enzym.,* 2006, vol. 40 (1-2), 8-15